# EUROPEAN PATENT APPLICATION

(11) **EP 4 679 370 A1**
(43) Date of publication of application: **14.01.2026**
(21) Application number: 25188850.9
(22) Date of filing: 10.07.2025
(51) Int. Cl.: G06T 7/73

(54) **MEDICAL IMAGE PROCESSING APPARATUS AND MEDICAL IMAGE PROCESSING METHOD**

(30) Priority: 10.07.2024 JP 2024110995
(71) Applicant: Canon Medical Systems Corporation, Tochigi 324-0036 (JP)
(72) Inventor: HIGAMI, Hirooki, Gifu, 500-8384 (JP); YAMAMOTO, Masanori, Gifu, 500-8384 (JP); AOYAMA, Gakuto, Otawara-shi, 324-0036 (JP); SAKAGUCHI, Takuya, Otawara-shi, 324-0036 (JP)
(74) Representative: Marks & Clerk LLP

(57) **Abstract**

According to one embodiment, a medical image processing apparatus includes an acquisition unit, a first region specifying unit, a first direction specifying unit, a second region specifying unit, a second direction specifying unit, and a feature amount calculation unit. The acquisition unit acquires a three-dimensional medical image of a patient requiring retainment of a prosthetic valve. The first direction specifying unit specifies a first direction of the three-dimensional medical image based on the first region of interest. The second direction specifying unit specifies a second direction of the three-dimensional medical image based on the second region of interest. The feature amount calculation unit calculates a first feature amount associated with the retainment based on the first direction and the second direction.

## Description

### FIELD

Embodiments described herein relate generally to a medical image processing apparatus and a medical image processing method.

### BACKGROUND

The aortic valve of the heart has three leaflets that open and close. The junctions (commissures) between the respective leaflets are separated at angular intervals of about 120°. Such an aortic valve is located between the left ventricle and the aorta of the heart. The leaflets open and close in accordance with the movement of the heart to make blood pass from the heart to the aorta. Such an aortic valve sometimes develops aortic stenosis, that is, the opening of the leaflets is restricted and narrowed due to various causes.

As a treatment method for aortic stenosis, transcatheter aortic valve implantation (TAVI) is known. In TAVI, a prosthetic valve that replaces the aortic valve of a patient is carried along a blood vessel by a catheter and is retained so as to almost overlap the position of the aortic valve. In a surgery procedure in the current TAVI, in order to retain a prosthetic valve at a proper angle, the prosthetic valve is inserted into a patient upon being rotated through a predetermined angle (currently, 90°) with respect to the patient and carried to a proper position by a catheter operation. Note that in a current surgery procedure, the same procedure is sometimes recommended even for patients having different anatomical structures based on the statistical analysis of preliminary clinical evaluation results.

According to studies by the present inventor on the same procedure described above, since, for example, the shape of the aortic valve associated with the positional relationship between the commissures differs for each patient, the proper retaining position of a prosthetic valve differs for each patient. Accordingly, there is room for improvement in a procedure for operating a catheter including the insertion angle of a prosthetic valve. That is, according to studies by the present inventor, in order to retain a prosthetic valve at a proper angle for each patient, a different operation procedure is required for each patient. It is therefore preferable to support for the operation procedure.

### SUMMARY

In relation to the embodiments, the following additional notes are disclosed as aspects and selective features of the invention.

(Additional Note 1) A medical image processing apparatus includes: an acquisition unit configured to acquire a three-dimensional medical image of a patient requiring retainment of a prosthetic valve; a first region specifying unit configured to specify a first region of interest from the three-dimensional medical image; a first direction specifying unit configured to specify a first direction of the three-dimensional medical image based on the first region of interest; a second region specifying unit configured to specify a second region of interest from the three-dimensional medical image; a second direction specifying unit configured to specify a second direction of the three-dimensional medical image based on the second region of interest; and a feature amount calculation unit configured to calculate a first feature amount associated with the retainment based on the first direction and the second direction.

(Additional Note 2) The first region of interest may include a first partial region and a second partial region different from each other. The first region specifying unit may be configured to specify the first direction based on a first positional relationship between the first partial region and the second partial region.

(Additional Note 3) The second region of interest may be part of the first region of interest.

(Additional Note 4) The second region of interest may be the same region as the first region of interest. The second region specifying unit may be configured to specify the second direction based on a second positional relationship between the first partial region and the second partial region.

(Additional Note 5) The first direction specifying unit may be configured to specify the first direction based on a position at which the first partial region and the second partial region indicated by the three-dimensional medical image overlap each other.

(Additional Note 6) The apparatus may further include: a third region specifying unit configured to specify a third region of interest different from the first region of interest and the second region of interest from the three-dimensional medical image; and a third direction specifying unit configured to specify a third direction of the three-dimensional medical image based on the third region of interest. The feature amount calculation unit may be configured to further calculate a second feature amount based on the first direction, the second direction, and the third direction.

(Additional Note 7) The first region of interest may be an aorta of the patient. The first partial region may be an ascending aorta of the patient. The second partial region may be a descending aorta of the patient. The second region of interest may be a commissure in the aortic valve of the patient. The third region of interest may be a coronary ostium. The first feature amount may be an angle at which the prosthetic valve is inserted into the patient. The second feature amount may be an angle obtained by correcting the first feature amount so as to implement retainment in a state in which the coronary ostium is not occluded.

(Additional Note 8) The feature amount calculation unit may be configured to calculate a third feature amount associated with the retainment based on a predetermined calculation formula using the first feature amount and the second feature amount as variables.

(Additional Note 9) The feature amount calculation unit may be configured to classify a shape of an anatomical structure in the first region of interest or the second region of interest, select a calculation formula corresponding to a result of the classification from a plurality of calculation formulae configured to calculate the first feature amount, and calculate the first feature amount based on the selected calculation formula.

(Additional Note 10) The feature amount calculation unit may be configured to calculate, as the first feature amount, an angle at which the prosthetic valve is inserted into the patient.

(Additional Note 11) The feature amount calculation unit may be configured to calculate, as the angle, a rotation amount of the prosthetic valve that rotates about an axis of a catheter holding the prosthetic valve.

(Additional Note 12) The angle may be an angle dependent on a position of a commissure of the aortic valve of the patient. The rotation amount may be a rotational angle for matching a commissure of the prosthetic valve with a commissure of the aortic valve.

(Additional Note 13) The apparatus may further include a display control unit configured to cause a display to display, side by side, a first display region that displays the three-dimensional medical image based on the first direction and a second display region that displays the three-dimensional medical image based on the second direction.

(Additional Note 14) The display control unit may be configured to update the first direction or the second direction in accordance with a user operation and update display contents on the display based on the updating result, and update the first feature amount based on the updating result.

(Additional Note 15) A medical image processing method includes: acquiring a three-dimensional medical image of a patient requiring retainment of a prosthetic valve; specifying a first region of interest from the three-dimensional medical image; specifying a first direction of the three-dimensional medical image based on the first region of interest; specifying a second region of interest from the three-dimensional medical image; specifying a second direction of the three-dimensional medical image based on the second region of interest; and calculating a first feature amount associated with the retainment based on the first direction and the second direction.

### BRIEF DESCRIPTION OF THE DRAWINGS

FIG. 1 is a block diagram showing an example of the arrangement of a medical image processing system including a medical image processing apparatus according to an embodiment.
FIG. 2 is a flowchart for explaining an operation according to the embodiment.
FIG. 3 is a schematic view for explaining an operation according to the embodiment.
FIG. 4 is a schematic view for explaining an operation in FIG. 3.
FIG. 5 is a schematic view for explaining an operation associated with the first region of interest according to the embodiment.
FIG. 6 is a schematic view for explaining an operation in FIG. 5.
FIG. 7 is a schematic view for explaining an operation in FIG. 5.
FIG. 8 is a schematic view for explaining an operation in FIG. 5.
FIG. 9 is a schematic view for explaining an operation associated with the second region of interest according to the embodiment.
FIG. 10 is a schematic view for explaining the first modification of the embodiment.
FIG. 11 is a schematic view for explaining the second modification of the embodiment.
FIG. 12 is a schematic view for explaining the second modification of the embodiment.
FIG. 13 is a schematic view for explaining the third modification of the embodiment.
FIG. 14 is a schematic view for explaining the fourth modification of the embodiment.
FIG. 15 is a schematic view showing an example of a menu in FIG. 14.
FIG. 16 is a schematic view showing an example of the menu in FIG. 14.
FIG. 17 is a schematic view showing an example of split screens in FIG. 14.
FIG. 18 is a schematic view showing an example of alignment display.
FIG. 19 is a schematic view showing an example of a setting screen in the menu in FIG. 16.

### DETAILED DESCRIPTION

In general, according to one embodiment, a medical image processing apparatus includes processing circuitry. The processing circuitry is configured to acquire a three dimensional medical image of a patient requiring retainment of a prosthetic valve. The processing circuitry is configured to specify a first region of interest from the three dimensional medical image. The processing circuitry is configured to specify a first direction of the three dimensional medical image based on the first region of interest. The processing circuitry is configured to specify a second region of interest from the three dimensional medical image. The processing circuitry is configured to specify a second direction of the three dimensional medical image based on the second region of interest. The processing circuitry is configured to calculate a first feature amount associated with the retainment based on the first direction and the second direction.

A medical image processing apparatus and a medical image processing method according to an embodiment will be described below with reference to the accompanying drawings. In the following description, the same reference numerals denote the constituent elements having almost the same functions and arrangements, and a repetitive description will be made as needed.

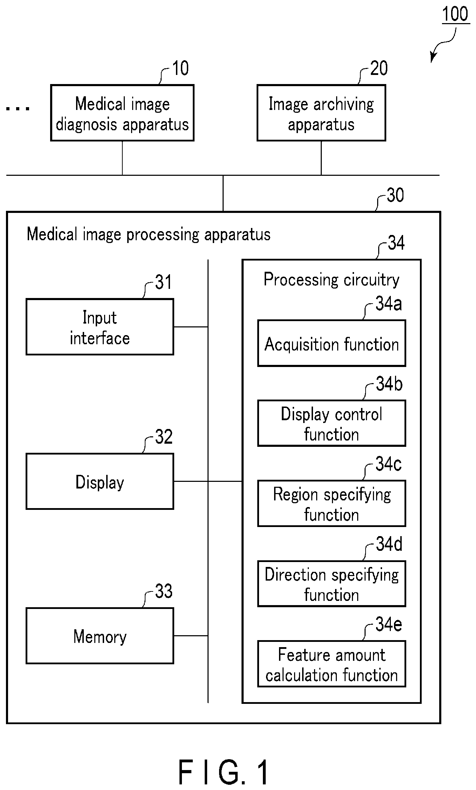

FIG. 1 is a view showing an example of the arrangement of a medical image processing system 100 according to an embodiment. The medical image processing system 100 includes a plurality of medical image diagnosis apparatuses 10, an image archiving apparatus 20, and a medical image processing apparatus 30. As shown in FIG. 1, the medical image diagnosis apparatus 10, the image archiving apparatus 20, and the medical image processing apparatus 30 are communicably connected to each other via a network wirelessly or wiredly. The network is, for example, a LAN (local area network). Note that as long as security is ensured by a VPN (virtual private network) or the like, a line to be connected is not limited to a LAN. In this case, the network is, for example, the Internet or a public communication line.

Note that the medical image processing system 100 may be implemented in the form of a thin client such that, for example, the client apparatus used by an operator is made to execute minimum necessary processing, and the server apparatus is made to execute most processing. At this time, the medical image processing apparatus 30 functions as a server apparatus. In addition, if the medical image processing system 100 is implemented in the form of a thin client, a client apparatus (not shown) is, for example, connected to the network as a terminal apparatus including an input interface, display, memory, and control function (to be described later). At this time, the terminal apparatus may function as, for example, a medical image display apparatus.

The plurality of medical image diagnosis apparatuses 10 image a patient and collect medical image data. The medical image diagnosis apparatuses 10 collect, for example, medical image data depicting blood vessels in patients. The medical image diagnosis apparatus transmits collected medical image data to the image archiving apparatus 20 and the medical image processing apparatus 30. For the sake of detailed description, assume that the plurality of medical image diagnosis apparatuses 10 are X-ray computed tomography (to be referred to as CT hereinafter) apparatuses. Note that the medical image diagnosis apparatus 10 is not limited to an X-ray CT apparatus and may be another type of imaging apparatus that can acquire medical image data, such as an X-ray diagnosis apparatus or a magnetic resonance imaging apparatus.

The X-ray CT apparatus collects medical image data concerning a patient by CT scanning on the patient. In this case, scan conditions are attached to medical image data. The CT scan conditions include, for example, an imaging target region, a scan scheme, a view count, a tube voltage, a tube current, and the body position (posture) of a patent at the time of the execution of CT scanning.

The image archiving apparatus 20 is an apparatus that archives medical image data collected by the medical image diagnosis apparatus 10. The image archiving apparatus 20 acquires medical image data from the medical image diagnosis apparatus 10 via the network and causes a memory provided in or outside the apparatus to store the acquired medical image data. For example, the image archiving apparatus 20 is implemented by computer equipment such as a server apparatus.

The medical image processing apparatus 30 acquires medical image data from the medical image diagnosis apparatus 10 or the image archiving apparatus 20 via a communication interface (not shown) and a network and executes various types of processing by using the acquired medical image data. The medical image processing apparatus 30 is implemented by, for example, a computer device such as a workstation. Note that the medical image diagnosis apparatus 10, the image archiving apparatus 20, and the medical image processing apparatus 30 can be installed in arbitrary places as long as they can be connected to each other via a network. For example, the medical image processing apparatus 30 may be installed in a facility, hospital, or the like different from that where the medical image diagnosis apparatus 10 is installed. In addition, the medical image processing apparatus 30 may be mounted on the medical image diagnosis apparatus 10 such as an X-ray diagnosis apparatus.

As shown in FIG. 1, the medical image processing apparatus 30 includes an input interface 31, a display 32, a memory 33, and processing circuitry 34.

The input interface 31 is implemented by a trackball, switches/buttons, a mouse, a keyboard, a touch pad (or track pad) that performs an input operation when the operator (user) touches the operation surface, a touch panel display (or touch screen) formed by integrating a display screen and a touch pad, and the like, which are used to input various types of instructions, commands, information, selections, and settings to the main body of the medical information processing apparatus from the operator (user). The input interface 31 is connected to the processing circuitry 34, converts an input operation received from the user into an electrical signal and outputs it to the processing circuitry 34. In this case, the input interface 31 may cause the display 32 to display a GUI (graphical user interface) that is used by the user to input various types of instructions using physical operation components such as a mouse and a keyboard. Note that in this specification, the input interface 31 is not limited to the one including physical operation components. For example, the input interface 31 includes processing circuitry that receives an electrical signal corresponding to an input operation from an external input device provided separately from the apparatus and outputs the electrical signal to the processing circuitry 34. In the following description, "an operation of the input interface 31 by the user" is also referred to as "a user operation".

The display 32 is constituted by a display main body that displays arbitrary data, internal circuitry that supplies a display signal to the display main body, and peripheral circuitry such as a connector or cable that connects the display main body to the internal circuitry. The display 32 displays various types of information under the control of the control function of the processing circuitry 34. For example, the display 32 displays various types of images generated by the processing circuitry 34. The display 32 also displays a GUI as the input interface 31. As the display 32, one of various types of arbitrary displays can be used as needed. For example, as the display 32, a liquid crystal display, organic EL (electro-luminescence) display, or plasma display can be used. The display 32 may be a desktop type display, a tablet terminal that can wirelessly communicate with the medical image processing apparatus 30, or the like.

The memory 33 is constituted by memories that record electrical information, such as a ROM (read only memory), RAM (random access memory), HDD (hard disk drive), and image memory, and peripheral circuitry such as memory controllers and memory interfaces accompanying the memories. The memory 33 stores, for example, various programs, such as a medical image processing program of the medical image processing apparatus 30, and various data, such as various tables, data during processing, and data after processing. Note that the medical image processing program is a program for making a computer function as the medical image processing apparatus 30 that executes a medical image processing method. The medical image processing program may be stored and distributed in a non-transitory computer readable storage medium and installed in the memory 33 upon being read out from the storage medium.

The processing circuitry 34 reads out medical image processing programs stored in the memory 33 based on the instruction input by the user via the input interface 31 and controls the medical image processing apparatus 30 in accordance with the read programs. For example, the processing circuitry 34 is a processor that implements the respective functions of the medical image processing apparatus 30 in accordance with the medical image processing programs read out from the memory 33. The respective functions include, for example, an acquisition function 34a, a display control function 34b, a region specifying function 34c, a direction specifying function 34d, and a feature amount calculation function 34e. Note that the respective functions may be implemented by being distributed to a plurality of processors. Alternatively, each function or part of each function may be implemented by another apparatus as needed.

The acquisition function 34a, the display control function 34b, the region specifying function 34c, the direction specifying function 34d, and the feature amount calculation function 34e as the respective functions of the processing circuitry 34 will be sequentially described next. Note, however, that the allocation of the respective functions described below is for convenience sake and can be changed as needed. Even if the processing allocated to a given function is allocated to another function, it is no different than the processing circuitry 34 executing the processing. That the allocation of the respective function can be changed applies to each embodiment and each modification.

The acquisition function 34a acquires a three-dimensional medical image of a patient in which a prosthetic valve needs to be retained. For example, the acquisition function 34a acquires a three-dimensional medical image including image information concerning the aortic valve of the patient. For example, the acquisition function 34a may acquire a three-dimensional medical image from either the medical image diagnosis apparatus 10 or the image archiving apparatus 20. In addition, for example, the acquisition function 34a may acquire a three-dimensional medical image from the memory 33 storing the three-dimensional medical image. The acquisition function 34a is an example of an acquisition unit.

The display control function 34b causes the display 32 to display data during processing by the processing circuitry 34 or as a processing result. For example, the display control function 34b may cause the display 32 to display the three-dimensional medical image. In addition, for example, the display control function 34b may cause the display 32 to display, side by side, the first display region for displaying a three-dimensional medical image based on the first display direction and the second display region for displaying a three-dimensional medical image based on the second display direction. In this case, the display control function 34b may update the first display direction or the second display direction in accordance with a user operation or update the display contents on the display 32 based on the updating result. The display control function 34b is an example of a display control unit.

The region specifying function 34c specifies the first region of interest from the three-dimensional medical image. In addition, the region specifying function 34c specifies the second region of interest from the three-dimensional medical image. The first region of interest is, for example, an image region of the first anatomical structure. The second region of interest is, for example, an image region of the second anatomical structure. A three-dimensional medical image is, for example, a medical image including image information concerning the aortic valve of a patient. Note that the image information of the aortic valve enables the aortic valve to be displayed as a sectional region of a three-dimensional medical image of the heart region. A supplementary explanation will be provided for an anatomical structure concerning the aortic valve. A general cardiac aortic valve has three leaflets (right coronary cusp, left coronary cusp, and non-coronary cusp) that open and close. The junctions (commissures) between the respective leaflets are separated at angular intervals of about 120°. The aortic valve is located between the left ventricle and the aorta of the heart. The leaflets open and close in accordance with the movement of the heart to control the bloodstream from the heart to the aorta. More specifically, the bloodstream from the left ventricle passes the aortic valve in the Valsalva sinus and sequentially flows through the ascending aorta, the aortic arch, and the descending aorta. In addition, part of the bloodstream passing the aortic valve flows to the left coronary artery and the right coronary artery through the two coronary ostiums in the Valsalva sinus. Note that the structure of the prosthetic valve retained inside the aortic valve has three leaflets (right coronary cusp, left coronary cusp, and non-coronary cusp) that open and close, similar to the anatomical structure of a general aortic valve, and the junctions (commissures) between the respective leaflets are separated at angular intervals of about 120°. The region specifying function 34c can specify, as a region of interest, an arbitrary structure of interest of the anatomical structure of such an aortic valve, such as the ascending aorta, the descending aorta, commissure, and coronary ostium, as needed. Note that the anatomical structure concerning the aortic valve, which has been roughly described above, is an example. However, not limited to this, and a known anatomical structure can be used as needed. Likewise, a region of interest is not limited to the above example of the structure of interest. The region specifying function 34c is an example of the first region specifying unit and the second region specifying unit.

The direction specifying function 34d specifies the first direction of a three-dimensional medical image based on the first region of interest. The direction specifying function 34d also specifies the second direction of a three-dimensional medical image based on the second region of interest. The "direction" in this case is a parameter that designates a two-dimensional image, of the two-dimensional images that can be displayed from a three-dimensional medical image, which is displayed on the display at the time of observing the three-dimensional medical image from a predetermined position. As a parameter corresponding to the "direction", for example, a display direction or a direction corresponding to an observation angle. Note, however, that as the "direction", an arbitrary parameter can be used as needed as long as it is a parameter specified based on a region of interest. The direction specifying function 34d is an example of the first direction specifying unit and the second direction specifying unit.

The feature amount calculation function 34e calculates the first feature amount concerning the retainment of a prosthetic valve base on the first direction and the second direction. For example, the feature amount calculation function 34e calculates the first feature amount concerning the shape of the aortic valve based on the first direction and the second direction. If the first direction or the second direction is updated, the feature amount calculation function 34e may update the display contents on the display 32 based on the updating result. The feature amount calculation function 34e may also calculate an angle at the time of inserting the prosthetic valve in a patient as the first feature amount. In addition, the feature amount calculation function 34e may calculate, as this angle, the rotation amount of the prosthetic valve that rotates about the axis of a catheter holding the prosthetic valve. In this case, the angle may be an angle dependent on the commissures of the aortic valve. In addition, the rotation amount may be a rotation angle for matching the commissures of the prosthetic valve with the commissures of the aortic valve. The feature amount calculation function 34e is an example of a feature amount calculation unit.

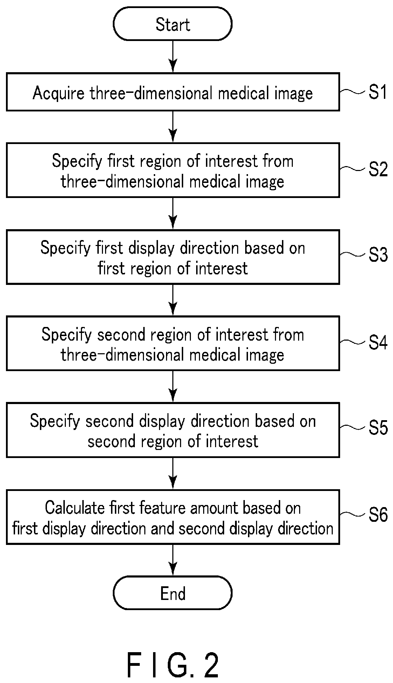

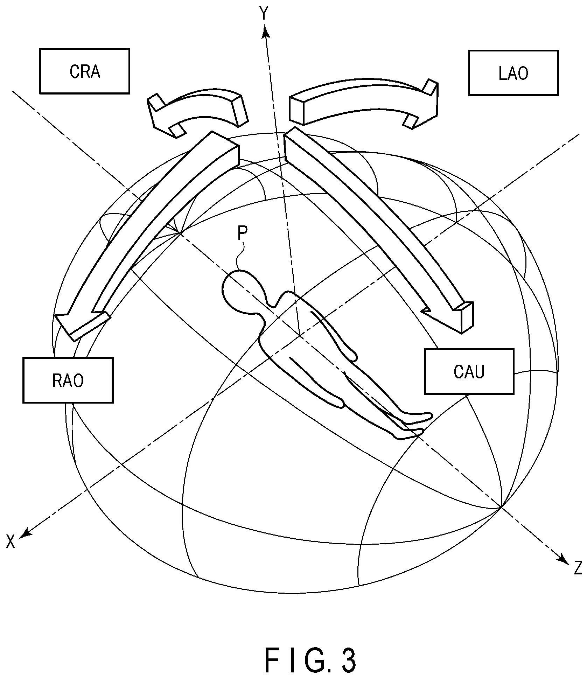

The operation of the medical image processing apparatus having the above arrangement will be described next with reference to the flowchart of FIG. 2 and the schematic views of FIGS. 3, 4, 5, 6, 7, 8, and 9. The following description will be made by exemplifying a case where the rotation amount of the prosthetic valve in TAVI is calculated based on the positional relationship between the ascending aorta and the descending aorta in a CT image and the positions of commissures of the aortic valve in the CT image. In addition, as the first direction, the second direction, and the third direction, the first display direction, the second display direction, and the third display direction will be respectively exemplified. Note, however, that the operation of the medical image processing apparatus 30 is not limited to this case.

### (Step S1)

The processing circuitry 34 of the medical image processing apparatus 30 causes the acquisition function 34a to acquire a three-dimensional medical image including image information concerning the aortic valve of a patient. For example, the processing circuitry 34 acquires a three-dimensional CT image of the patient from the image archiving apparatus 20. Note that any type of three-dimensional medical images may be used as long as it is a type that stores the form information of the three-dimensional anatomical structure of a target living tissue. For example, a three-dimensional medical image may be a medical image obtained by another imaging apparatus, such as an ultrasonic image, MRI image, X-ray image, PET image, or SPECT image or part of four-dimensional image obtained by capturing a three-dimensional medical image a plurality of times in the time direction. Note that the operation in step S1 may be started by an instruction from the user or automatically started when a new medical image is archived during monitoring of the image archiving apparatus 20 such as a PACS. Alternatively, the processing circuitry 34 may determine whether the new medical image satisfies a predetermined condition, and the processing may be executed if the condition is satisfied. The condition may be any condition that can determine the state of an image. For example, the processing may be executed under the condition whether the new medical image is the one obtained by imaging the heart according to an imaging protocol designed for the heart or under the condition whether the new medical image is the one which is enlarged/reconstructed according to a reconstruction method. In addition, the processing circuitry 34 may execute the processing if a combination of these conditions is satisfied.

### (Step S2)

The processing circuitry 34 causes the region specifying function 34c to specify the first region of interest from the three-dimensional medical image. More specifically, the processing circuitry 34 specifies the region indicated by the first living organ of interest in the acquired CT image as the first region of interest. That is, according to the embodiment, although the coordinate information of each pixel indicated by the aorta on the CT image is specified, a region indicated by any living organ may be specified. Note that the aorta as the specified first region of interest includes both the ascending aorta and the descending aorta. As specific processing, the position of the first region of interest may be specified to specify the region by the operation of the input interface 31. Alternatively, the first region of interest may be specified based on the anatomical structure depicted in a CT image by a known region extraction technique. A known region extraction technique is, for example, the Otsu's method based on CT values, a region expansion method, the snake method, the graph cut method, or a mean shift method. Note that for the processing in step S2, it is possible to use an arbitrary method of specifying the first region of interest from a medical image. For example, the first region of interest may be specified by using a shape model of a region of interest constructed based on learning data prepared in advance using a machine learning technique (including deep learning). In addition, the execution of the above processing such as the graph cut method with respect to an entire medical image tends to take too much cost. Accordingly, a region (to be referred to as an associated region hereinafter) that is associated with the first living organ of interest and is larger than the first region of interest and smaller than the entire medical image may be specified, and the above processing is applied to only the associated region to specify the first region of interest. If, for example, the first living organ of interest is the aorta, a heart region, a region around the mediastinum, or the like corresponds to the associated region. Note that an associated region may be set by the operation of the input interface 31. If the aorta is specified as the first region of interest, the ascending aorta and the descending aorta included in the aorta may be specified as separate regions. In this case, the above processing may be separately applied to an associated region of the ascending aorta and an associated region of the descending aorta. Alternatively, the aorta specified as the first region of interest may be separated into the ascending aorta region and the descending aorta region based on a feature amount (for example, curvature) of the aorta. Note that in the processing in step S2, the first region of interest has an arbitrary size, and, for example, the coordinates of one point (for example, one pixel) may be specified as the first region of interest.

### (Step S3)

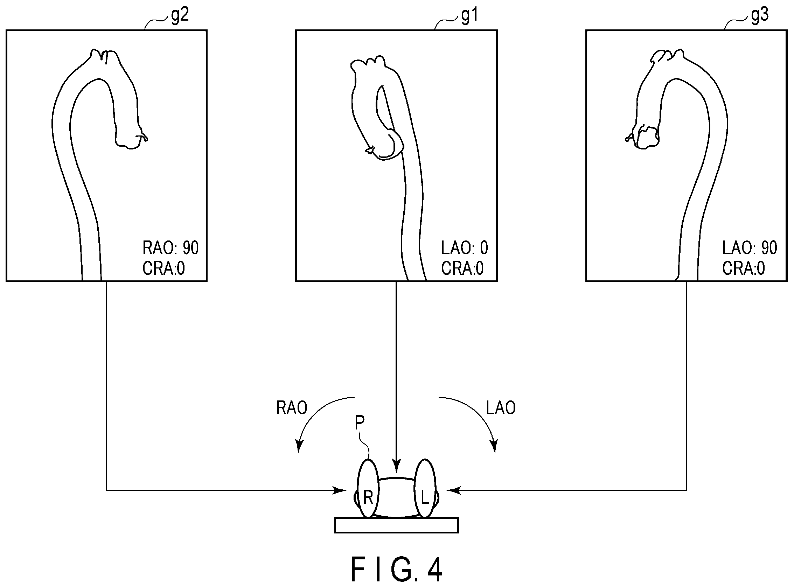

The processing circuitry 34 causes the direction specifying function 34d to specify the first display direction of the three-dimensional medical image based on the first region of interest. In this case, the first display direction is a direction in which a three-dimensional anatomical structure of the patient is observed from a predetermined direction and the anatomical structure is two-dimensionally displayed on the display 32. As shown in FIG. 3, this example uses a coordinate system in a state in which a patient P is placed on the top in a supine position. The coordinate system is a clinical coordinate system in which the imaging central position of the patient P is the origin. In the clinical coordinate system, an axis parallel to the body axis of the patient P is the Z-axis, an axis parallel to the front direction of the patient P is the Y-axis, and an axis orthogonal to the Z-axis and the Y-axis is the X-axis. As shown in FIG. 3, the rotation angle of the patient P to the right-hand side around the Z-axis as a rotation axis, with the position of the Y-axis along the front direction of the patient P being a reference position, is represented by the first oblique position (Right Anterior Oblique view to be referred to as RAO hereinafter), and the rotation angle of the patient P to the left-hand side is represented by the second oblique position (Left Anterior Oblique view to be referred to as LAO hereinafter). The rotation angle of the patient P to the head side around the X-axis as a rotation axis, with the position of the Y-axis being a reference position, is represented by a head direction (CRAnial to be referred to as CRA hereinafter), and the rotation angle of the patient P to the foot side is represented by a caudal direction (CAUdal to be referred to as CAU hereinafter). The first display direction is designated by a combination of these directions. That is, LAO (or RAO) 0° and CRA (or CAU) 0° represent an angle at which the patient P is observed from the front. In addition, RAO 90° and CRA 0° represent an angle at which the patient P is observed from a position tilted from the front to the right-hand side by 90°. LAO 90° and CRA 0° represent an angle at which the patient P is observed from a position tilted from the front to the left-hand side by 90°. FIG. 4 exemplarily shows medical images g1 to g3 displaying the aorta as the first region of interest from the directions corresponding to these observation angles.

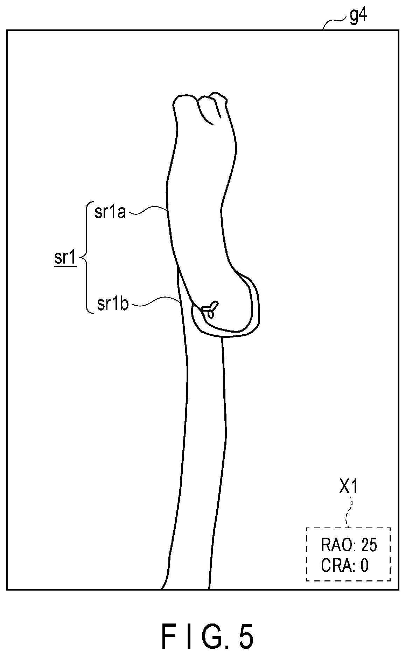

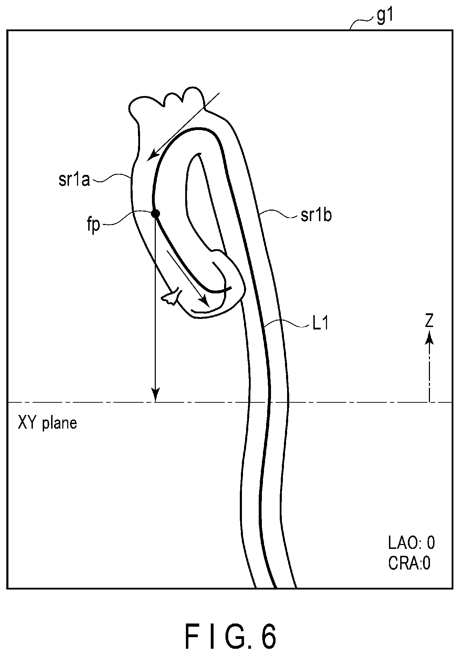

In step S3, the processing circuitry 34 specifies the first display direction based on the positional relationship between the ascending aorta and the descending aorta included in the aorta as the first region of interest. For example, the processing circuitry 34 specifies the first display direction based on a position at which the ascending aorta and the descending aorta indicated by the three-dimensional medical image associated with the front direction of the patient overlap each other. More specifically, as shown in FIG. 5, the angle defined by CRA 0° and RAO 25° at a position at which a descending aorta sr1b and an ascending aorta sr1a overlap each other is specified as a first display direction X1 from a medical image g4 indicating an aorta srI. Note that the first display direction X1 in the medical image g4 is specified as shown in, for example, FIGS. 6, 7, and 8.

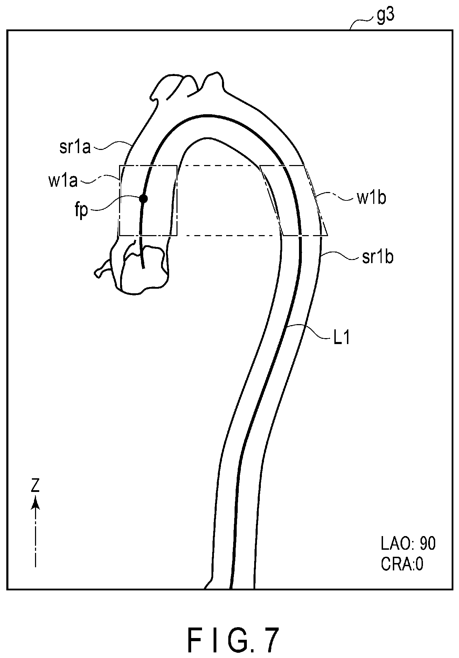

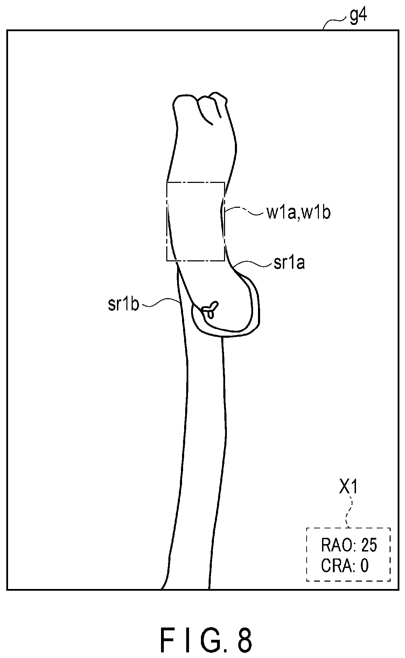

As shown in FIG. 6, the processing circuitry 34 obtains, as a feature point fp, one of points on an axial line L1 within the range of ascending aorta sr1a in the front medical image g1, at which a tangent line to the axial line L1 is most perpendicular to an XY plane. More specifically, the processing circuitry 34 obtains, as the feature point fp, a point at which the Z component (the component in the Z-axis direction) of a unit vector on the tangent line is largest. Subsequently, as shown in FIG. 7, the processing circuitry 34 calculates a region w1a of the ascending aorta sr1a and a region w1b of the descending aorta sr1b in the range of widths of several cm in the positive and negative Z-axis directions centered on the feature point fp. Subsequently, as shown in FIG. 8, the processing circuitry 34 rotates the ascending aorta sr1a and the descending aorta sr1b with respect to the Z-axis and specifies the first display direction X1 from the rotation angle at which the two regions w1a and w1b overlap most each other. Note that if there are a plurality of rotation angles at which the two regions overlap most each other, the angle specified first may be used. In addition, the two regions w1a and w1b need to overlap each other while the ascending aorta sr1a is located on the near side (front side) with respect to the descending aorta sr1b. The rotation center may be the center of gravity of the aorta. According to another method, the processing circuitry 34 may specify a point on the descending aorta sr1b at which the Z-coordinate is the same as the feature point fp and specify the first display direction X1 from the angle defined by CRA 0° and RAO at which the feature point fp and a point on the descending aorta sr1b overlap each other. Note that the first display direction X1 is not limited to CRA 0° and RAO 25° shown in FIG. 5 and FIG. 8, and an arbitrary direction can be used as long as it is the direction specified based on the first region of interest. In addition, the first display direction X1 may be specified by any method. For example, the first display direction X1 may be specified by specifying an angle at which the descending aorta sr1b and the ascending aorta sr1a overlap most each other by changing the display direction of a three-dimensional VR (Volume Rendering) image using the VR image display function of a known medical image display apparatus. With a known VR image display function, it is necessary to set a rotation center for changing the display direction, and the rotation center can be set at an arbitrary position. For example, a rotation center may be set at a point associated with the first region of interest (for example, the center of gravity). Alternatively, the amount of overlapping between the descending aorta sr1b and the ascending aorta sr1a may be calculated in various arbitrary directions (for example, 360 directions in increments of 1° up to 360°) so as to specify, as the first display direction X1, a direction in which the amount of overlapping becomes largest. In this case, the processing circuitry 34 may calculate the amount of overlapping between the entire regions of the ascending aorta sr1a and the descending aorta sr1b or, as described above, calculate the amount of overlapping between only partial regions of the ascending aorta sr1a and the descending aorta sr1b. For example, the processing circuitry 34 may extract the axial lines L1 of the ascending aorta sr1a or the descending aorta sr1b based on a known technique, calculate feature amounts at positions on the axial line L1, and control a region in which an overlapping position is to be calculated based on the feature amounts. For example, the processing circuitry 34 may obtain, as feature amounts, curvatures at the respective positions on the axial line L1 which is substantially a curve or obtain, as feature amounts, the directions calculated at the respective positions on the axial line L1 by first derivation. Note that in the processing in step S3, an arbitrary method can be used as long as it can specify the first display direction X1 based on the first region of interest and is not limited to any method of specifying directions. For example, the first display direction X1 may be specified by using a learning model constructed based on learning data prepared in advance using a machine learning technique (including deep learning).

### (Step S4)

The processing circuitry 34 causes the region specifying function 34c to specify the second region of interest from the three-dimensional medical image. More specifically, the processing circuitry 34 specifies the region indicated by the second living organ of interest in the acquired CT image as the second region of interest. In this case, the processing circuitry 34 extracts the coordinate information of a pixel indicating the commissure between the right coronary cusp and the left coronary cusp of the aortic valve included in the CT image by a known region extraction method and specifies the region of the pixel indicating the commissure as the second region of interest. Alternatively, the processing circuitry 34 may specify, as the second region of interest, the coordinate information of pixels indicating the commissure between the right coronary cusp and the non-coronary cusp and the commissure between the left circuitry and the non-coronary cusp of the aortic valve included in the CT image. Note that the known region extraction method is the same as that described with reference to step S2. Note that in step S2, the first region of interest and the second region of interest may be specified in parallel, or the second region of interest may be specified regarding the first region of interest as an associated region of the second region of interest. Alternatively, the second region of interest may be specified by using any another method.

### (Step S5)

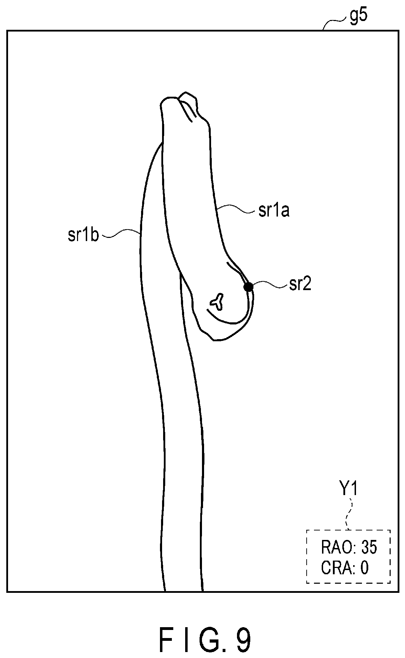

The processing circuitry 34 causes the direction specifying function 34d to specify the second display direction of the three-dimensional medical image based on the second region of interest. For example, the processing circuitry 34 specifies the second display direction based on the positions of the commissures of the aortic valve indicated by the three-dimensional medical image. More specifically, the processing circuitry 34 specifies the second display direction based on the positions (coordinate information) of the commissures specified in step S4. That is, as shown in FIG. 9, the processing circuitry 34 specifies, as a second display direction Y1, the angle defined by CRA 0° and RAO 35° at which a commissure sr2 is located on the most right-hand side from the medical image g5 indicating the ascending aorta sr1a and the descending aorta sr1b. Alternatively, the processing circuitry 34 specifies, as the second display direction Y1, the angle defined by CRO 0° and the RA at which the Y-coordinate of the pixel indicated by the commissure between the right coronary cusp and the non-coronary cusp is the same as that of the pixel indicated by the commissure between the left coronary cusp and the non-coronary cusp. Note that the second display direction Y1 is not limited to this, and an arbitrary direction can be used as long as it is set based on the second region of interest. In addition, steps S2 and S3 may be executed after steps S4 and S5.

### (Step S6)

The processing circuitry 34 causes the feature amount calculation function 34e to calculate the first feature amount associated with the shape of the aortic valve based on the first display direction and the second display direction. For example, the processing circuitry 34 calculates, as the first feature amount, the angle at which a prosthetic valve is to be inserted into a patient so as to retain the prosthetic valve at an angle matching the shape. More specifically, for example, the processing circuitry 34 calculates, as the angle of the insertion, the rotation amount of a prosthetic valve rotating about the axis of a catheter holding the prosthetic valve. That is, the processing circuitry 34 calculates, as a correction amount for a rotation amount θ, the difference obtained by subtracting the angle indicated by the second display direction Y1 from an angle (2X) twice the angle indicated by the first display direction X. This correction amount is the one for 90° which is the rotation angle currently used to match the commissures of the prosthetic valve with the commissures of the aortic valve. That is, the rotation amount θ as a manipulated variable in commissure alignment (commissure alignment) for adjusting the retainment position of the prosthetic valve, focusing on the commissures, corresponds to θ = 90 - (2X - Y1). Note, however, that the rotation amount θ is not limited to the calculation formula of θ = 90 - (2X - Y1). The rotation amount θ can be calculated by an arbitrary calculation formula based on the first display direction X and the second display direction Y1.

As described above, according to the embodiment, the processing circuitry 34 acquires a three-dimensional medical image including image information associated with the aortic valve of the patient as a three-dimensional medical image of the patient requiring the retainment of a prosthetic valve. The processing circuitry 34 specifies the first region of interest from the three-dimensional medical image. The processing circuitry 34 specifies the first display direction X in the three-dimensional medical image based on the first region of interest. The processing circuitry 34 specifies the second region of interest from the three-dimensional medical image. The processing circuitry 34 specifies the second display direction Y1 in the three-dimensional medical image based on the second region of interest. The processing circuitry 34 calculates the first feature amount associated with the shape of the aortic valve as the first feature amount associated with retainment based on the first display direction X and the second display direction Y1. The arrangement configured to calculate the first feature amount associated with the retainment of a prosthetic valve from a three-dimensional medical image of a patient who requires the retainment of the prosthetic valve enables the first feature amount for each patient to be used for surgery. This makes it possible to support surgery so as to retain a prosthetic valve at a proper angle for each patient.

According to the embodiment, the processing circuitry 34 may specify the first display direction X based on the position at which the ascending aorta and the descending aorta indicated by a three-dimensional medical image associated with the front direction of a patient overlap each other. The processing circuitry 34 may specify the second display direction Y1 based on the positions of the commissures of the aortic valve indicated by the three-dimensional medical image. With this operation, the arrangement using positions that can be easily specified from a three-dimensional medical image in addition to the above effect can relatively easily specify the first display direction X and the second display direction Y1.

According to the embodiment, the processing circuitry 34 may calculate an angle at which a prosthetic valve is to be inserted into a patient as the first feature amount. In this case, in addition to the above effect, it is possible to directly support surgery.

According to the embodiment, the processing circuitry 34 may calculate, as an angle at which a prosthetic valve is to be inserted, the rotation amount of the prosthetic valve rotated about the axis of the catheter holding the prosthetic valve. In this case, in addition to the above effect, it is possible to more directly support surgery. For example, even if the manipulated variable of the catheter differs from the rotation amount of the prosthetic valve, the actual rotation amount of the prosthetic valve can be used for surgery.

In addition, according to the embodiment, an angle at which a prosthetic valve is to be inserted may be an angle dependent on the commissures of the aortic valve of a patient. In addition, the rotation amount may be the one for matching the commissures of the aortic valve with the commissures of the prosthetic valve. In this case, in addition to the above effect, it is possible to support surgery so as to almost match the positions of the commissures of the prosthetic valve with the positions of the commissures of the aortic valve for each patient.

### (First Modification)

In the embodiment, two display directions are specified from two regions of interest. However, not limited to this, two display directions may be specified from one region of interest. For example, the processing circuitry 34 may cause the region specifying function 34c to specify the first region of interest and the second region of interest as the same region and specify the first display direction and the second display direction from the same region. For example, the second region of interest may be the same region as the first region of interest. In this case, the first region of interest may include the first partial region and the second partial region which are different from each other. Accordingly, the processing circuitry 34 may cause the direction specifying function 34d to specify the first display direction X based on the first positional relationship between the first partial region and the second partial region. The second region of interest may be part of the first region of interest. The first positional relationship may be a relationship in which the first partial region and the second partial region are located at a position at which they overlap most when the first region of interest is rotated about an arbitrary rotation center and observed from a predetermined direction. The processing circuitry 34 may cause the direction specifying function 34d to specify the second display direction Y1 based on the second positional relationship between the first partial region and the second partial region. The second positional relationship may be a relationship in which the contours of the first partial region and the second partial region partially come into contact with each other when the first region of interest is rotated about an arbitrary rotation center and observed from a predetermined direction. The first positional relationship and the second positional relationship each are preferably a positional relationship set when a three-dimensional medical image associated with the front direction (CRA (or CAU) 0°) of a patient is observed from a predetermined RAO (or LAO) direction. However, the embodiment is not limited to the front direction of the patient.

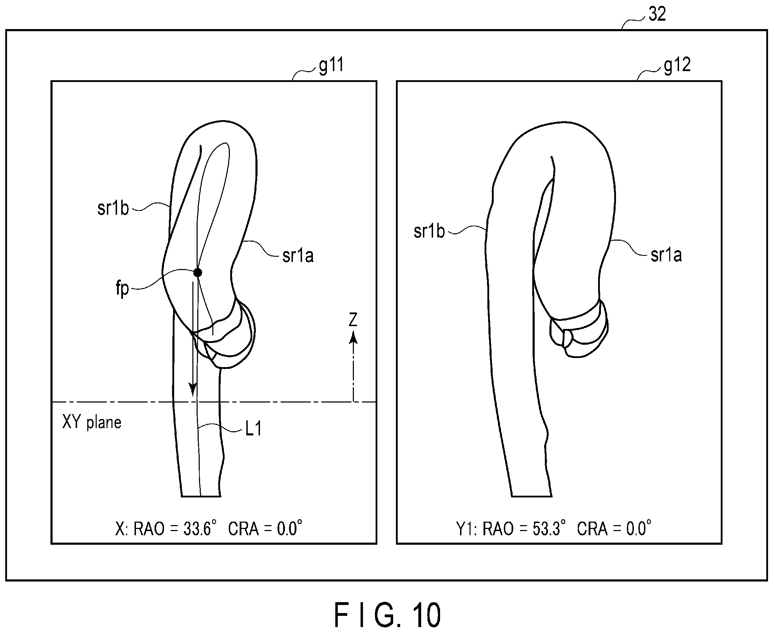

FIG. 10 is a schematic view for explaining the first modification of the embodiment. Referring to FIG. 10, a medical image g11 used to specify the first display direction X and a medical image g12 used to specify the second display direction Y1 are displayed on the display 32. That is, the processing circuitry 34 causes the display control function 34b to make the display 32 display, side by side, the first display region that displays the medical image g11 based on the first display direction X and the medical image g12 based on the second display direction Y1. The medical images g11 and g12 are three-dimensional medical images. The first partial region is the ascending aorta sr1a. The second partial region is the descending aorta sr1b.

The medical image g11 indicates an example of the first positional relationship in which the ascending aorta sr1a and the descending aorta sr1b are located at a position where they overlap when the aorta is observed from a direction from the front side to the rear side of the display 32 upon rotation of the first region of interest about the center of gravity of the aortic valve as a rotation center. In this case, the medical image g11 indicates the first positional relationship in which the feature point fp, of the respective points on the axial line L1 in the ascending aorta sr1a, at which a tangent line to the axial line L1 is most perpendicular to an XY plane overlaps the axial line L1 in the descending aorta sr1b. Note, however, that the first positional relationship is not limited to this and may, for example, indicate the position at which the amount of overlapping between the region indicated by the ascending aorta sr1a and the region indicated by the descending aorta sr1b becomes largest in the observation direction. In the embodiment, the processing circuitry 34 causes the direction specifying function 34d to specify the angle defined by CRA 0° and RAO 33.6° as the first display direction X based on the first positional relationship between the ascending aorta sr1a and the descending aorta sr1b, as indicated by the medical image g11.

The medical image g12 indicates an example of the second positional relationship in which the contours of the ascending aorta sr1a and the descending aorta sr1b are located at positions at which they partially come into contact with each other when the aorta is observed from a direction from the front side to the rear side of the display 32 upon rotation of the first region of interest about the center of gravity of the aortic valve as a rotation center. Note that the second positional relationship may be referred to as a relationship in which the ascending aorta sr1a and the descending aorta sr1b do not overlap but are located closest to each other. In any case, the processing circuitry 34 causes the direction specifying function 34d to specify, as the second display direction Y1, the angle defined by CRA 0° and RAO 53.3° based on the second positional relationship between the ascending aorta sr1a and the descending aorta sr1b.

The processing circuitry 34 calculates the first feature amount based on a calculation formula different from that described above in accordance with such modifications of the first display direction X and the second display direction Y1. As such a calculation formula for such first feature amount, a formula matching the modified first and second display directions X and Y1 is set in advance in the feature amount calculation function 34e.

According to the first modification described above, the first region of interest (the aorta sr1) includes the first partial region (the ascending aorta sr1a) and the second partial region (the descending aorta srlb). The second region of interest is identical to the first region of interest. The processing circuitry 34 specifies the first display direction X based on the first positional relationship between the ascending aorta sr1a and the descending aorta sr1b. The processing circuitry 34 specifies the second display direction Y1 based on the second positional relationship between the ascending aorta sr1a and the descending aorta sr1b. In addition to the effect of the embodiment, this makes it possible to reduce the processing of specifying the second region of interest. In the first modification, as well as in the case shown in FIG. 10, an arbitrary arrangement can be used as needed as long as it is configured to specify two display directions from one region of interest.

According to the first modification, the first positional relationship is a relationship in which the ascending aorta sr1a and the descending aorta sr1b are located at positions at which they overlap most in a predetermined observation direction. The second positional relationship is a relationship in which the contours of the ascending aorta sr1a and the descending aorta sr1b are located at positions at which they partially overlap in a predetermined observation direction. Accordingly, in addition to the above effect, it is possible to specify two display directions based on two positional relationships that are easily visually checked.

### (Second Modification)

In the embodiment and the first modification, one feature amount (the first feature amount) is calculated from the same or different regions of interest. However, not limited to this, and two or more feature amounts (the first feature amount, the second feature amount,...) may be calculated from three or more regions of interest. For example, the processing circuitry 34 causes the region specifying function 34c to specify the third region of interest different from the first region of interest and the second region of interest from a three-dimensional medical image. In addition, for example, the processing circuitry 34 causes the direction specifying function 34d to specify the third display direction in a three-dimensional medical image based on the third region of interest. Accordingly, the processing circuitry 34 may cause the feature amount calculation function 34e to further calculate the second feature amount associated with the retainment of a prosthetic valve based on the first display direction, the second display direction, and the third display direction. In this case, the processing circuitry 34 may cause the feature amount calculation function 34e to calculate the third feature amount associated with retainment based on a predetermined calculation formula using the first feature amount and the second feature amount as variables. For example, the processing circuitry 34 may calculate the third feature amount associated with the retainment of a prosthetic valve based on a predetermined calculation formula using the first feature amount and the second feature amount as variables. The region specifying function 34c is an example of a third region specifying unit. The direction specifying function 34d is an example of a third direction specifying unit.

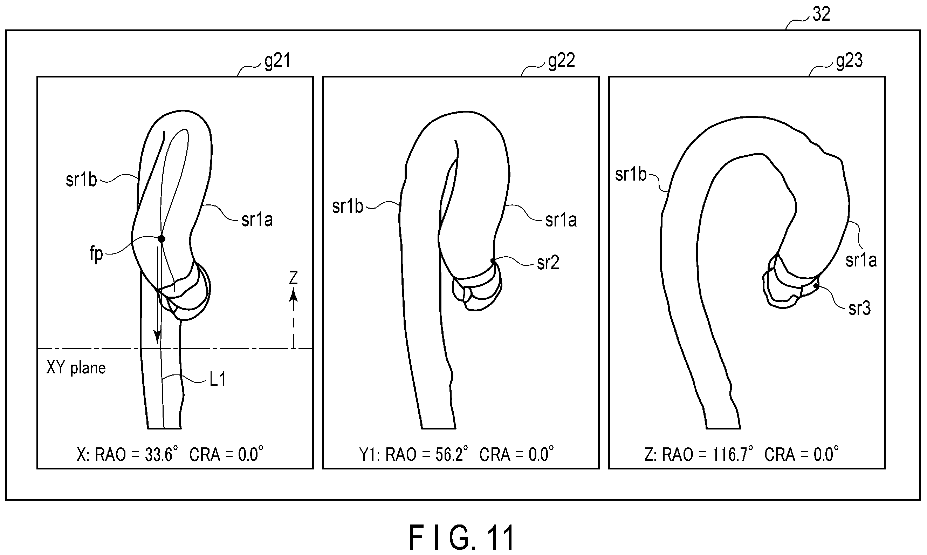

FIG. 11 is a schematic view for explaining the second modification of the embodiment. Referring to FIG. 11, the display 32 displays a medical image g21 used to specify the first display direction X, a medical image g22 used to specify the second display direction Y1, and a medical image g23 used to specify a third display direction Z. The medical images g21 to g23 are three-dimensional medical images. The first region of interest is the aorta sr1 including the ascending aorta sr1a and the descending aorta sr1b. The second region of interest is the commissure sr2 between the right coronary cusp and the left coronary cusp of the aortic valve. The third region of interest is a coronary ostium sr3 located between the ascending aorta sr1a and aortic valve.

In the medical image g21, as in the first modification, the angle defined by CRA 0° and RAO 33.6° is specified as the first display direction X based on the first positional relationship in which the ascending aorta sr1a and the descending aorta sr1b overlap.

In the medical image g22, as in step S5 in the embodiment, the angle defined by CRA 0° and RAO 56.2° at which the commissure sr2 is located on the most right-hand side is specified as the second display direction Y1. Note that since the medical image g22 in the second modification and the medical image g5 in step S5 in the embodiment are medical images of different patients, even if each commissure sr2 is located on the most right-hand side, the RAO values are different from each other.

In the medical image g23, the angle defined by CRA 0° and RAO 116.7° at which the coronary ostium sr3 is located on the most right-hand side is specified as the third display direction Z.

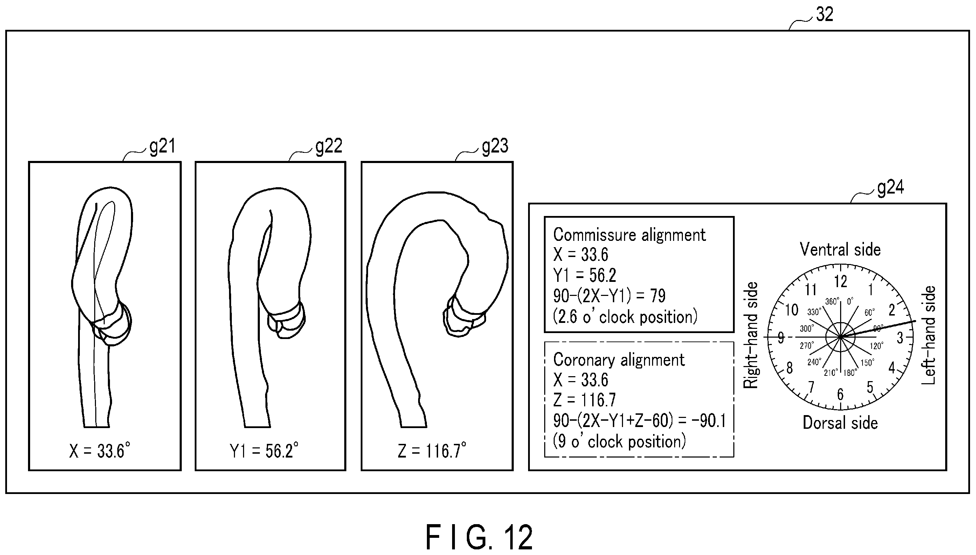

As indicated by an image g24 in FIG. 12, the processing circuitry 34 executes coronary alignment for adjusting the retainment position of a prosthetic valve, focusing on the coronary ostium, in addition to the above commissure alignment, to further calculate the second feature amount associated with the shape of the aortic valve based on the first display direction X, the second display direction Y1, and the third display direction Z. For example, the processing circuitry 34 sets, as a correction amount for a rotation amount ϕ as the second feature amount, the difference obtained by subtracting 60° from the sum of the angle indicated by the third display direction Z and the difference obtained by subtracting the angle indicated by the second display direction Y1 from the angle (2X) twice the angle indicated by the first display direction X. Note that 60° used in this case originates is derived from the fact that the commissures of the aortic valve are shifted from each other by about 120°. However, the calculation formula may be changed to calculate and use the angles formed by the commissures in the respective patients. This correction value is the one from 90° as the rotation angle currently used to retain a prosthetic valve so as not to occlude the coronary artery (coronary ostium) branched from the aorta. That is, the rotation amount ϕ as a manipulated variable in coronary alignment for adjusting the retainment position of a prosthetic valve, focusing on the coronary ostium, corresponds to ϕ = 90 - (2X - Y1 + Z - 60). Note that the rotation amount ϕ may be expressed as θ + Z - 60 using the rotation amount ϕ as a manipulated variable in commissure alignment. In this case, the rotation amount ϕ in coronary alignment is an example of the angle obtained by correcting the rotation amount ϕ in commissure alignment. To elaborate further, the rotation amount ϕ in commissure alignment is an example of the first feature amount as an angle at which a prosthetic valve is to be inserted into a patient. The rotation amount ϕ in coronary alignment is an example of the second feature amount as the angle obtained by correcting the first feature amount so as to retain a prosthetic valve in a state in which the coronary ostium is not occluded. Note that the rotation amount ϕ is not limited to the above calculation formula. The rotation amount ϕ can be calculated by an arbitrary calculation formula based on the first display direction X, the second display direction Y1, and the third display direction Z.

Alternatively, the processing circuitry 34 may calculate the third feature amount (Ω) associated with the shape of the aortic valve based on a predetermined calculation formula using the first feature amount (θ) and the second feature amount (Ω) as variables. It is possible to use, as the predetermined calculation formula, for example, a formula (Ω = θ + ϕ) for adding the first feature amount (θ) and the second feature amount (ϕ), a formula (Ω = θ - ϕ) for subtracting the second feature amount (ϕ) from the first feature amount (θ), or a formula (Ω = (θ + ϕ)/2) for averaging the first feature amount (θ) and the second feature amount (ϕ), as needed.

According to the second modification described above, the processing circuitry 34 specifies the third region of interest (the coronary ostium sr3) different from the first region of interest (the aorta sr1) and the second region of interest (the commissure sr2) from the three-dimensional medical image. The processing circuitry 34 specifies the third display direction Z in the three-dimensional medical image based on the third region of interest. The processing circuitry 34 further calculates the second feature amount based on the first display direction X, the second display direction Y1, and the third display direction Z. with this operation, in addition to the effect of the embodiment, the arrangement configured to calculate the second feature amount other than the first feature amount can support surgery.

According to the second modification, the first region of interest is the aorta including the ascending aorta and the descending aorta of the patient. The second region of interest is a commissure in the aortic valve of the patient. The third region of interest is the coronary ostium between the ascending aorta and the aortic valve. The first feature amount is an angle at which a prosthetic valve is to be inserted into the patient. The second feature amount is the angle obtained by correcting the first feature amount so as to retain a prosthetic valve in a state in which the coronary ostium is not occluded. In this case, in addition to the effect of the embodiment, the arrangement configured to calculate an angle by correcting the first feature amount so as to retain a prosthetic valve in a state in which the coronary ostium is not occluded can elaborately support surgery.

According to the second modification, the processing circuitry 34 may calculate the third feature amount associated with the shape of the aortic valve based on a predetermined calculation formula using the first feature amount and the second feature amount as variables. In this case, in addition to the above effect, the arrangement configured to calculate the third feature amount other than the first feature amount and the second feature amount can support surgery. According to the second modification, other than the examples shown in FIGS. 11 and 12, an arbitrary arrangement can be used as needed as long as it is configured to calculate two or more feature amounts from three or more regions of interest.

### (Third Modification)

The embodiment has exemplified the methods of specifying a display direction based on the feature amount of the axial line L1 in step S3, specifying a display direction based on the position of the commissure sr2 in step S5, and calculating the first feature amount in step S6, based on predetermined specifying methods. However, the scope of the embodiment is not limited to this. For example, the processing circuitry 34 may classify the shapes of anatomical structures in the first region of interest and the second region of interest, select a calculation formula to be applied to the calculation of a feature amount based on the classifying result from a plurality of predetermined calculation formulae that can calculate the first feature amount, and calculate the first feature amount based on the selected calculation formula. That is, the processing circuitry 34 may change the display direction specifying method in steps S3 or S5 or the calculation formula used in step S6 based on the first region of interest and the second region of interest. For example, the processing circuitry 34 may execute the determination processing of classifying the shape of a region of interest before step S3 or S5. In the determination processing, the processing circuitry 34 calculates the feature amount of the shape of the anatomical structure of the first region of interest or the second region of interest, classifies the shape by comparing the shape with a predetermined decision criterion, and determines the feature amount to be calculated based on the classifying result, the position of the region of interest, or the calculation formula to be applied. More specifically, for example, if a target anatomical structure is the aortic valve, the processing circuitry 34 may extract the aortic valve as the first region of interest or the second region of interest, calculate the number of leaflets of the aortic valve as the feature amount of the shape, and perform determination in accordance with the number of leaflets. Although the number of leaflets of the aortic valve is normally three, the number of leaflets is sometimes two (a bicuspid aortic valve) or four (quadricuspid aortic valve) depending on the disease type. In this case, the calculation formula used in step S6 is preferably changed in accordance with the number of leaflets. As the feature amount of the shape, the feature amount calculated from only the shape of a region of interest such as the size of the valve ring or a relative feature such as the distance from an arbitrary third region of interest different from the region of interest may be used. Alternatively, the shape feature of an arbitrary third region of interest different from the region of interest may be used. If, for example, the region of interest is the aortic valve, the calculation formula may be changed in accordance with the curvature (substantially linear shape, substantially curved shape, or substantially arc shape) of the ascending aorta as the third region of interest.

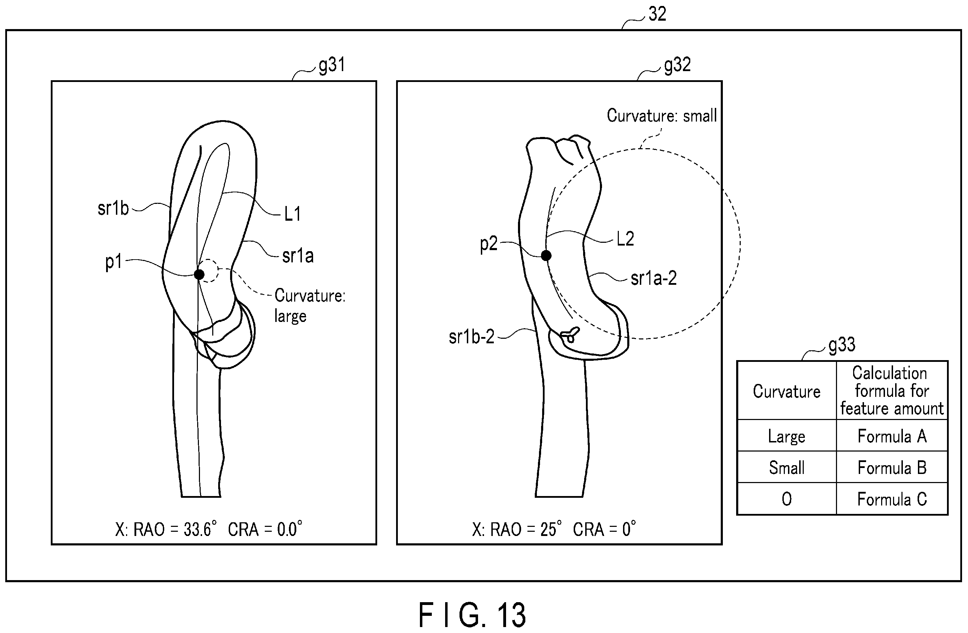

FIG. 13 is a schematic view for explaining the third modification of the embodiment. Referring to FIG. 13, the display 32 displays a medical image g31 indicating the ascending aorta sr1a and the axial line L1 of a given patient and a medical image g32 indicating an ascending aorta sr1a-2 and an axial line L2 of another patient. In addition, the medical images g31 and g32 also indicate the descending aorta sr1b and a descending aorta srlb-2 partially hidden by the ascending aorta sr1a and the ascending aorta srla-2. In the medical images g31 and g32 of the different patients, curvatures at the respective positions on the axial line L1 of the ascending aorta sr1a and the axial line L2 of the ascending aorta sr1a-2 may be obtained as feature amounts. In this case, a calculation formula to be applied may be determined in accordance with the curvature. For example, as indicated by a schematic view g33, the range of curvatures and feature amount calculation formulae may be set in advance, and a calculation formula may be determined in accordance with the curvatures at points p1 and p2 on the axial lines L1 and L2. In this case, if the curvature at the point p1 on the axial line L1 is large, formula A is determined as a calculation formula to be used. If the curvature at the point p2 on the axial line L2 is small, formula B is determined as a calculation formula to be used. Note that if the curvature is 0, formula C is determined as a calculation formula to be used. In addition, the calculation formula may be changed in accordance with the amount of calcification of the aortic valve. That is, the processing circuitry 34 calculates the feature amount associated with an anatomical structure for each patient, determines the feature amount based on a predetermined decision criterion, and changes the calculation formula or the like to be applied to each patient in accordance with the determination result.

According to the third modification described above, the processing circuitry 34 classifies the shape of the anatomical structure in the first region of interest or the second region of interest, selects a calculation formula corresponding to the classifying result from a plurality of calculation formulae that can calculate the first feature amount, and calculates the first feature amount based on the selected calculation formula. With this operation, in addition to the effect of the embodiment, it is possible to calculate the first feature amount based on the calculation formula in accordance with the classifying result on the shape of an anatomical structure for each patient.

### (Fourth Modification)

Although the display contents are not described in detail in the embodiment, a specific example of the display contents on the display 32 will be described in detail. Accordingly, the processing circuitry 34 causes the display control function 34b to update the first display direction X or the second display direction Y1 in accordance with the operation by the operator and update the display contents on the display 32 based on the updating result. The processing circuitry 34 causes the feature amount calculation function 34e to update the first feature amount based on the updating result. That is, the processing circuitry 34 updates (recalculates) the first feature amount associated with the shape of the aortic valve based on the updated first display direction X and the updated second display direction Y1.

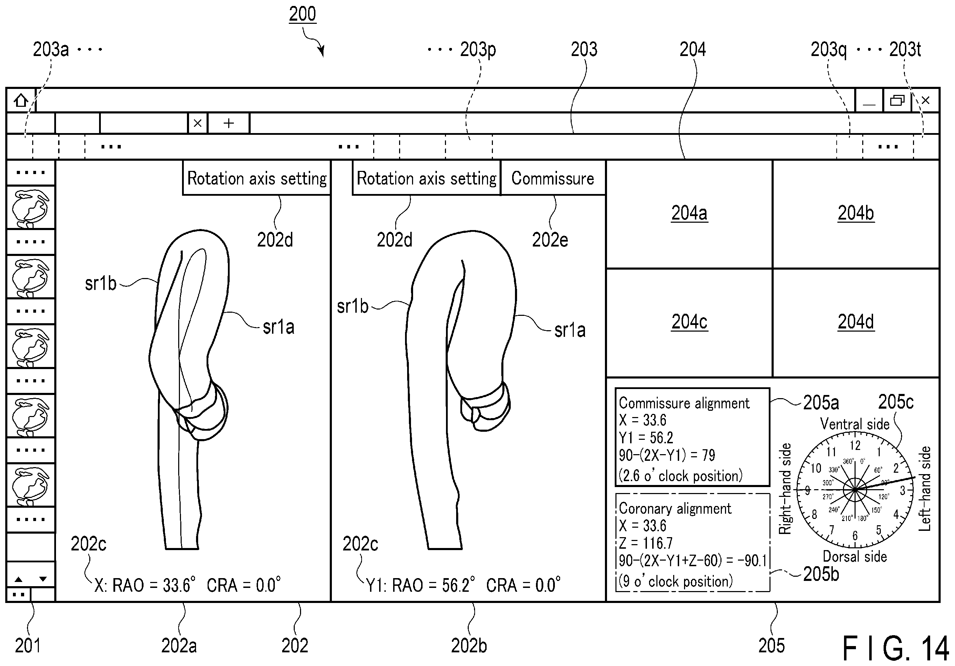

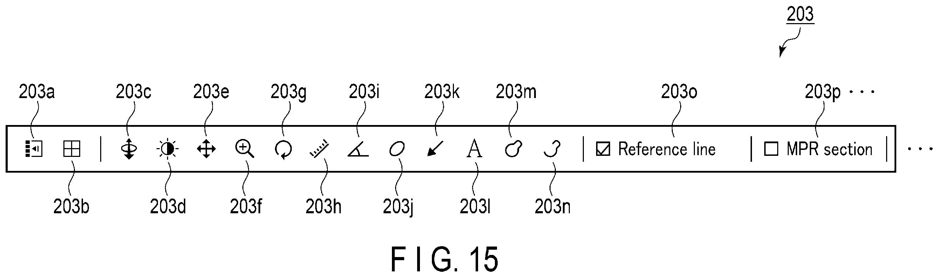

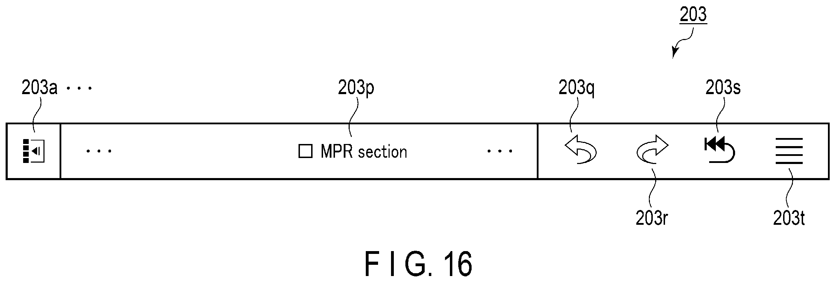

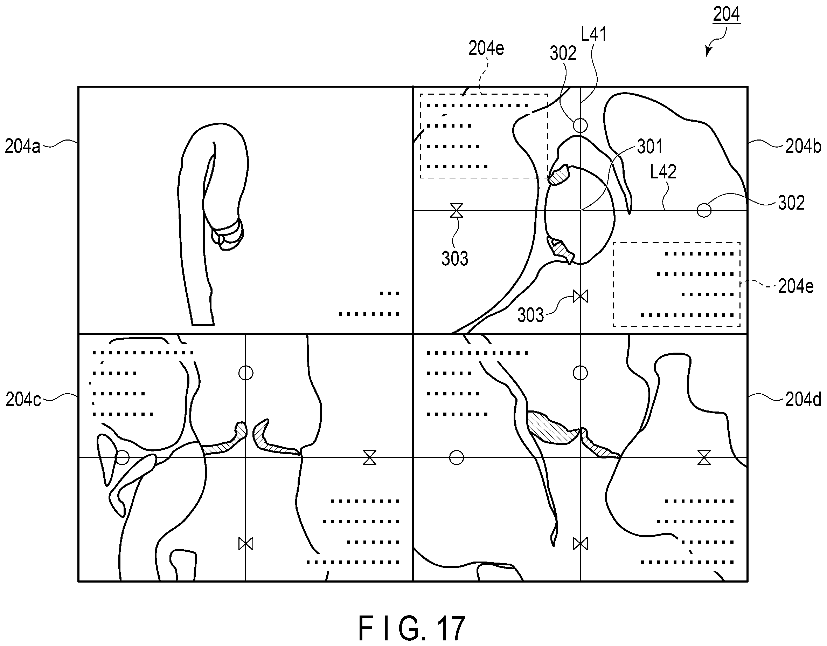

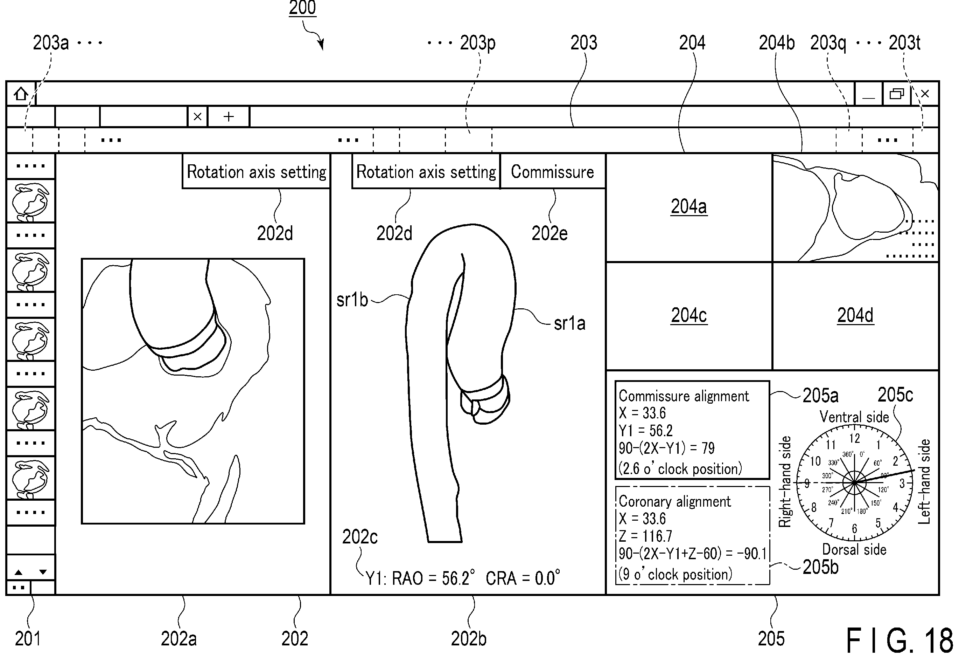

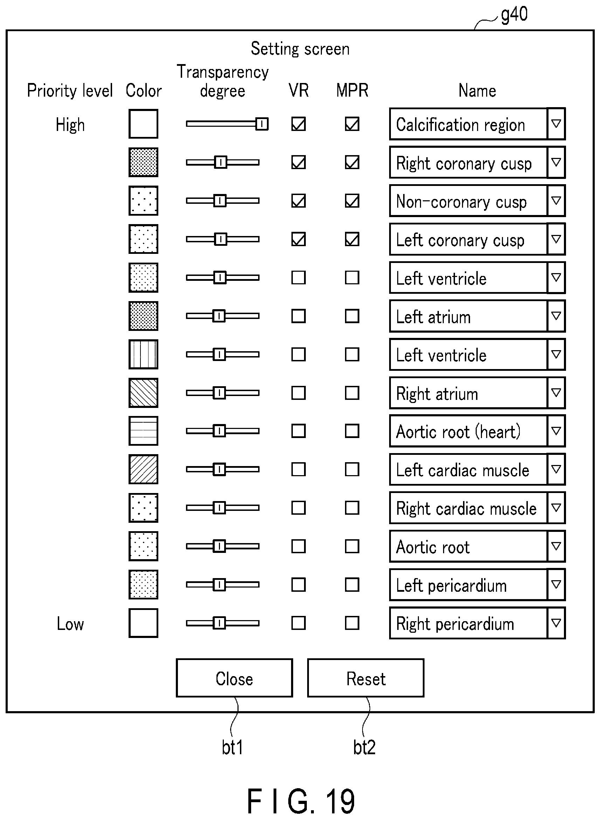

FIG. 14 is a schematic view for explaining the fourth modification of the embodiment. FIGS. 15 and 16 are schematic views showing an example of a menu. FIG. 17 is a schematic view showing an example of a split screen. FIG. 18 is a schematic view showing an example of positioning display. FIG. 19 is a schematic view showing an example of a setting screen on the menu.

As shown in FIG. 14, a display screen 200 of the display 32 includes, for example, a thumbnail region 201, a medical image region 202, a menu bar region 203, a split image region 204, and a feature amount calculation region 205.

In this case, the thumbnail region 201 is a display region of thumbnail images and displays a list of images acquired from an image database or an examination apparatus in a hospital based on an instruction from the user. The images displayed in the list are images that satisfy the conditions designated by the user using the input interface 31 that designates image conditions (not shown). The conditions can designate information associated with a patient as an object in an image, such as the name, ID, birth date, and weight of the patient, information associated with the image, such as the modality type of the image, imaging apparatus name, imaging date, imaging conditions, and reconstruction conditions, and the like. These pieces of information can be acquired from the DICOM (digital imaging and communication in medicine) header of the image, PACS (picture archiving and communication system), electronic chart, RIS (radiology information system), HIS (hospital information system), and the like. The following is a description of an example in which one CT image of a predetermined patient is designated. However, images of a plurality of patients and images (for example, CT images and ultrasonic images) obtained by different types of modality may be designated. The thumbnail region 201 displays icons indicating images that satisfy the designated conditions. Each icon to be used may be the reduced image obtained by reducing a two-dimensional image of a representative section of a designated image in accordance with the size of the thumbnail region 201 or a character string or symbol representing the designated image. Alternatively, the icons to be used may be various figures, images, and schema images archived in advance in a storage device. In addition, the thumbnail region 201 may display, side by side, an icon and the basic information (an imaging date, slice count, reconstruction function, and the like) in the designated image. Basic information to be displayed may be set in advance or designated by the user. These pieces of basic information can be acquired from the DICOM header of an image, PACS, electronic chart, RIS, HIS, and the like. The user selects an icon displayed in the thumbnail region 201 and drags and drops the icon in the medical image region 202 or the split image region 204, thereby displaying an image corresponding to the icon in the medical image region 202 or the split image region 204. If a medical image has already been displayed in the medical image region 202 at the time of drag and drop operation, a warning (not shown) may be displayed to the user to remove the displayed medical image from the medical image region 202, and a medical image corresponding to the dragged and dropped icon may be displayed afterward. Note that drag and drop operation may be called a moving operation or copying operation depending on what is performed.

The split image region 204 is a display region of various images. The split image region 204 is split into display regions 204a to 204d that are random in count and size and respectively displays sectional images at predetermined positions in acquired medical images or the processed images calculated by applying image processing to the medical images in the display regions 204a to 204d. Note that the display regions 204a to 204d are examples and are random in count and size. Images are respectively displayed in the display regions 204a to 204d under different display conditions associated with each other. The types of images and the display conditions are set in advance. The display conditions include the manner of assigning images, for example, designating what kinds of images should be displayed in which of the display regions 204a to 204d, cross-section positions, enlargement ratios, window levels, and window widths in displaying sectional images, and the like.

The images displayed in the medical image region 202 and the split image region 204 or in the display regions 202a, 202b, 204a, 204b, 204c, and 204d can be displayed upon changing of the observation sections, a slice feed (browse), or changing of enlargement ratios, central positions (translation), window levels, widow widths, and the like. In addition, the sizes of the display regions 202a, 202b, 204a, 204b, 204c, and 204d can be changed. For example, the sizes of the display regions 202a, 202b, 204a, 204b, 204c, and 204d can be changed by, for example, a drag and drop operation or the like for the frames of the respective display regions or a specific operation on the respective display regions. As the specific operation, for example, double clicking or clicking while pressing on the Ctrl key can be used as needed. In addition, information set in advance or designated by the user is superimposed and displayed at a specific position on one of the display regions 202a, 202b, 204a, 204b, 204c, and 204d (for example, the position of an end 204e of the display region 204b). As the information, it is possible to designate information associated with a patient, such as the name, ID, birth date, and weight of the patient, information associated with the image, such as the modality type of the image, imaging apparatus name, imaging date, imaging conditions, and reconstruction conditions, and the like. These pieces of information can be acquired from the DICOM header of the image, PACS, electronic chart, RIS, HIS, and the like.

As shown in FIGS. 14, 15, and 16, the menu bar region 203 displays a menu bar including buttons 203a, 203b, 203q, 203r, 203s, and 203t, icons 203c to 203n, and checkboxes 203o and 203p. The user selects one of the icons by operating the input interface 31 such as a mouse and activates a function assigned to one of the buttons, the icon, or one of the checkboxes. The processing circuitry 34 causes the display control function 34b to update the display contents of the display 32 in accordance with the operation of the menu bar region 203 by the user.

The button 203a is a GUI for switching between display and non-display of the thumbnail region 201. The processing circuitry 34 hides the thumbnail region 201 in accordance with an operation on the button 203a and enlarges at least one of the medical image region 202, the split image region 204, and the feature amount calculation region 205 in accordance with the size of the hidden thumbnail region 201.

The button 203b is a GUI for changing the division count in the medical image region 202 or the split image region 204. Referring to FIGS. 14 and 17, the split image region 204 is divided into 2 rows x 2 columns, namely, the display regions 204a to 204d. However, the number of rows or columns of the split region can be changed. In addition, the sizes of the display regions 202a, 202b, 204a, 204b, 204c, and 204d may be able to be changed. Sets of division counts and sizes of the display regions 202a, 202b, 204a, 204b, 204c, and 204d in several patterns may be registered in advance as presets, and the display regions 202a, 202b, 204a, 204b, 204c, and 204d in a pattern corresponding to a designated set may be set. In addition, the user may be enabled to register new presets.

The icons 203c to 203g are an icon group of functions of assigning mouse operation sequences. For example, selecting a given icon will perform control to assign the operation sequence of left-clicking and dragging the mouse to an operation sequence corresponding to the selected icon.

More specifically, the icon 203c can assign the browse operation sequence of continuously displaying an image in the slice direction to the operation sequence of left-clicking and dragging the mouse.

The icon 203d can assign the operation sequence of changing the gray level of an image (WL or WW in CT) to the operation sequence of left-clicking and dragging the mouse.

The icon 203e can assign the operation sequence of translating an image to the operation sequence of left-clicking and dragging the mouse.

The icon 203f can assign the operation sequence of changing the enlargement ratio of an image to the operation sequence of left-clicking and dragging the mouse.

The icon 203g can assign the operation sequence of rotating an image to the operation sequence of left-clicking and dragging the mouse. This is not exhaustive, and the icons 203c to 203g may be respectively assigned to the operation sequence of right-clicking and dragging the mouse, the operation sequence of clicking and dragging the mouse wheel, and the operation sequence of simultaneously right- and left-clicking and dragging the mouse.

The icons 203c to 203g may be configured to set a scrolling speed or amount corresponding to a browsing function with respect to the movement amount of the mouse, a change amount of enlargement ratio, a movement amount of translation, a change amount of gray level, and a rotation amount.

The assignment may be changed in accordance with a mouse operation at the time of selecting one of the icons 203c to 203g. If, for example, one of the icons is selected by left-clicking, control may be performed to assign an operation sequence corresponding to the icon to the operation sequence of left-clicking. If one of the icons is selected by right-clicking, control may be performed to assign an operation sequence corresponding to the icon to the operation sequence of right-clicking. If one of the icons is selected by simultaneous right- and left-clicking, control may be performed to assign an operation sequence corresponding to the icon to the operation sequence of simultaneous right- and left-clicking. If one of the icons is selected by mouse wheel clicking, control may be performed to assign an operation sequence corresponding to the icon to the operation sequence of mouse wheel clicking.

The icons 203h to 203n are an icon group corresponding to drawing and measuring functions for various figures. Control may be performed to enable the implementation of drawing and measuring functions for various figures by selecting icons from the icons 203h to 203n.

More specifically, the icon 203h is a GUI for drawing a straight line on an image and measuring and displaying the length of the straight line. Note that it is possible by a user operation to adjust a display mode concerning the positions of the start and end points, color, and thickness of the straight line, the font of a measurement value, and the like.

The icon 203i is a GUI for drawing two straight lines on an image and measuring and displaying the acute angle formed by the two straight lines. Note that it is possible by a user operation to adjust a display mode concerning the positions of the start and end points, color, and thickness of each straight line, the font of a measurement value, and the like.

The icon 203j is a GUI for drawing an ellipse on an image and measuring and displaying the perimeter of the ellipse, the area of the ellipse, and the statistic (the average value, maximum value, minimum value, or the like) of the pixel values inside the ellipse. Note that it is possible by a user operation to adjust a display mode concerning the central position, major axis, minor axis, color, and thickness of the ellipse, the font of a measurement value, and the like.

The icon 203k is a GUI for drawing an arrow icon on an image. Note that it is possible by a user operation to adjust a display mode concerning the positions of the start and end points of the arrow icon, the color and thickness of the arrow icon, the form of the distal end portion, and the like.

The icon 2031 is a GUI for displaying an arbitrary character string on an image and enabling the user to designate the character string to be displayed by using a keyboard and the like. Note that it is possible by a user operation to adjust a display mode concerning the position at which the character string is to be displayed, the font and the background color of the character string, and the like.

The icon 203m is a GUI for drawing a closed curve having an arbitrary shape on an image and calculating and displaying the perimeter of the closed curve, the area of the closed curve, and the statistic (the average value, maximum value, minimum value, or the like) of the pixel values inside the closed curve. Note that it is possible by a user operation to adjust a display mode concerning the central position, color, and thickness of the closed curve, the font of a measurement value, and the like. In addition, this icon may be configured to set a predetermined shape (a circle, ellipse, rectangle, square, triangle, or the like) as the closed curve and adjust the length of each side of the set shape, the angle formed by two sides, the diameter, major radius, minor radius, or the like of the shape, and draw the shape of a free form.

The icon 203n is a GUI for drawing a closed curve having an arbitrary shape on an image and calculating and displaying the perimeter of the closed curve. Note that it is possible by a user operation to adjust a display mode concerning the central position, color, and thickness of the closed curve, the font of a measurement value, and the like. In addition, control may be performed to set a three-dimensional figure (a sphere, oval sphere, rectangular parallelepiped, rectangular pyramid, or the like) and calculate and display the surface area, volume, and the like of the set three-dimensional figure.

The checkbox 203o is a GUI for switching between display and non-display of a reference line indicating a cross-section position of an image displayed in another split region on an image. Selecting the checkbox 203o makes it possible to perform the above switching operation. Referring to FIG. 17, applying operations to reference lines L41 and L42 allows the user to change the display modes of the images displayed in the display regions 204c and 204d. That is, the processing circuitry 34 causes the display control function 34b to update the display contents on the display 32 in accordance with operations on the reference lines L41 and L42 by the user. Likewise, the processing circuitry 34 causes the display control function 34b to update the display contents on the display 32 in accordance with operations on an intersection 301, a circular mark 302, and a triangular mark 303 of the reference lines L41 and L42 by the user.

For example, the processing circuitry 34 can move the intersection 301 by selecting the intersection 301 or its surrounding portion between the reference lines L41 and L42 and dragging and dropping the selected portion in accordance with a user operation. If the intersection 301 is moved, the cross-section positions indicated by the reference lines L41 and L42 also change. Accordingly, the sectional images displayed in the display regions 204c and 204d also change. In addition, translating the reference lines L41 and L42 by dragging and dropping allows the user to change the sectional images in the display regions 204c and 204d in accordance with the changes in the cross-section positions indicated by the reference lines L41 and L42. Furthermore, rotating the reference lines L41 and L42 about the intersection 301 by dragging and dropping allows the user to change the sectional images in the display regions 204c and 204d in accordance with the changes in the cross-section positions indicated by the reference lines L41 and L42. Note that in rotating the reference lines L41 and L42, control may be performed to make one reference line operate in conjunction with the operation of the other reference line so as to always keep the angle constant between the reference lines L41 and L42. The formed angle may be the one obtained by equally dividing 180° based on the number of reference lines. In this case, if there are two reference lines, the formed angle is 90° due to vertical intersection. If there are three reference lines, the formed angle is 60°. Alternatively, in rotating the reference lines L41 and L42, each reference line may be independently moved. In translating and rotating operations, the user may switch between rotation by an operation on the circular mark 302 on the reference lines L41 and L42 in the display regions 204b to 204d and translation by an operation on the reference line L41 or L42 outside the circular mark 302. Alternatively, the user may use the keyboard to switch between translation and rotation of the reference lines L41 and L42. In using the keyboard, for example, the user may switch between rotation by a pressing operation on the Ctrl key and translation by releasing (non-contact) of the Ctrl key. In addition, the user may designate a width by using the reference lines L41 and L42, generate maximum intensity projection images or minimum intensity projection images in the range based on the designated width, and display the projection images in the display regions 204c and 204d corresponding to the reference lines L41 and L42. The operation of designating a width may be the operation of moving one of the two triangular marks 303 having corresponding vertices overlapping each other on the reference lines L41 and L42 away from a corresponding one of the reference lines L41 and L42. In this case, an Mip image can be generated with respect to the sectional image indicated by the range between the vertices of the two triangular marks 303 on the reference line L41 side and the reference line L42 side. In addition, the display mode of the reference lines L41 and L42 (color, thickness, and line type (solid line, dotted line, fineness of the dotted line, and the like)) may be set in advance or designated by the user.

The checkbox 203p is a GUI for switching whether to display an arbitrary two-dimensional sectional image upon aligning it with an image indicating a three-dimensional region, such as a rendering image, for example, a VR image or SR (Surface Rendering) image. For example, as shown in FIG. 18, the sectional image in the display region 204b is displayed in the display region 202a, with the three-dimensional position of the sectional image being associated with the VR image of the aorta displayed in the display region 202b. In superimposing the images, the VR image located in front of the section in the observation direction is displayed, but the VR image located at the back side of the section is not displayed. For a sectional image to be superimposed, the user may be able to designate a display region or split region. For example, the user may specify each display region by designating it using the context menu displayed by right-clicking on the display region. Alternatively, a display region (for example, the display region 204b) as a section to be superimposed may be set in advance, and the section displayed in the display region may be superimposed. In this case, in superimposing images, although the cross-section position and the size are matched with the position and enlargement ratio of the VR image, other display conditions (window conditions and the like) may be matched with the display conditions displayed in the split region. As these conditions alone, preset conditions may be used as fixed conditions. If, for example, the user operates on the display region in which a superimposed sectional image is displayed while the checkbox 203p is set, the display conditions for the sectional image also change in conjunction with the user operation or the like. If, for example, display conditions (scrolling and the like) are changed by a user operation or the like, the display conditions for the sectional image superimposed on the VR image also change accordingly.

The buttons 203q to 203s are GUIs for restoring the states of the medical image region 202 and the split image region 204 to specific states upon selection. The specific states include, for example, the state at the startup of an application (initial state), the state set when an image is displayed for the first time in a display region, and the state before a predetermined number of operations (for example, a return button (undo button), an advance button (redo button), and an image of a reset button). Referring to FIG. 16, the button 203q is a return button, and the button 203r is an advance button. The button 203s is a reset button. In any case, the buttons 203q to 203s are implemented by recording display conditions or display modes in specific states and restoring the recorded contents. Alternatively, display conditions or modes in a predetermined period may be recorded, and operating the buttons 203q and 203r may allow display states immediately before and after a given state to be continuously restored.

The button 203t is a GUI for displaying a setting screen for setting display conditions for a region to be superimposed on an image indicating a three-dimensional region, such as a rendering image like a VR image or SR image, or a two-dimensional image. FIG. 19 shows an example of a setting screen g40. The processing circuitry 34 causes the display control function 34b to update the display contents on the display 32 in accordance with an operation on the setting screen g40 by the user. Note that the position information of each type of region is specified in step S2. The setting screen g40 will be described later.

A combo box for designating "Name" is used to set a display region in which display conditions are to be changed. "Priority Level" indicates that higher priority levels are assigned to regions designated closer to the top of the screen. If a plurality of regions correspond to the same coordinates on an image, regions having higher priority levels are displayed.

The column of "Color" displays color samples assigned to the regions (designated from the combo box of "Name") in the corresponding column when they are superimposed on a VR image or a sectional image. As shown in FIG. 19, the user can change the color of a given region to an arbitrary color by selecting a region indicating a color sample. A given color may be changed by designating an RBG value or designating a color from a color map.

The column of "Transparency degree" enables the user to set a transparency degree in superimposed display of a region (designated from the combo box "Name") of the corresponding column on a VR image or a sectional image. A transparency degree can be designated by each slider bar from 0% to 99% in increments of 1%. If the transparency degree is 0%, superimposed display is performed without any transparency (that is, no background image can be seen).

Although not shown, display conditions such as a saturation and luminosity may be set, and textures and the like may be set in place of colors. In addition, selecting the "linked" checkbox (not shown) may make it possible to set transparency degrees of all the regions. In a coordinating operation, control may be performed to set all transparency degrees to the same value or to increase or decrease transparency degrees as a whole while maintaining the relationship between the values of the transparency degrees corresponding to the respective regions at the time of selection of a checkbox.

The checkboxes in the column of "VR" are GUIs for designating regions to be displayed on a VR image.

The checkboxes in the column of "MPR" are GUIs for designating regions to be displayed on an MPR (Multi Planar Reconstruction) image.

Although not shown, a button may be set so as to simultaneously check or cancel all the checkboxes. In addition, the combo boxes in the column of "Name" can designate regions to be displayed according to set priority levels and display conditions. Note that control may be performed so as not to set the same region in a plurality of combo boxes. If, for example, the user designates, in a given combo box, the region that has already been set in another combo box, control may be performed to inhibit the designation, or the existing combo box setting may be canceled. Alternatively, if the same region is set, control may be performed to preferentially use a setting with a higher priority level.

A close button bt1 is a GUI for hiding the setting screen g40.

A reset button bt2 is a GUI for restoring the set state of the setting screen g40 to the initial state. In addition, the timing at which the display conditions set by the setting screen g40 are reflected in the display region may be the timing immediately after each condition is set or the timing corresponding to an operation on the close button bt1.

The display regions 202a and 202b are, for example, regions in which the first region of interest and the second region of interest are displayed. The display regions 202a and 202b are used to specify the first display direction X and the second display direction Y1 of regions of interest in steps S3 and S5. The display directions of the VR images displayed in the display regions 202a and 202b are used as the first display direction X and the second display direction Y1 specified in steps S3 and S5. The initial display directions in the display regions 202a and 202b may be RAO and CRA or may be the display directions calculated by a predetermined method. The region 202c displays the first display direction X and the second display direction Y1 corresponding to the display directions of VR images.

The display regions 202a and 202b respectively include the regions 202c and the button 202d. The display region 202b further includes the button 202e.

The display of the region 202c is controlled to be changed in conjunction with a change in the display direction of the VR image in the region 202c by the user.

The button 202d is a GUI for fixing an arbitrary rotation axis when the display direction of a VR image is changed and can arbitrarily fix each of the X-, Y-, and Z-axes. With the function of the button 202d, a VR image can be rotated in only a specific direction. For example, the button 202d is used to change only the rotational angle of RAO while CRA is fixed. That is, the button 202d is operated to perform control to change the display direction only if the mouse is operated in a predetermined direction and to perform control so as not to change the display direction if the mouse is operated in other directions. A rotation axis to be fixed may be an axis based on a patient coordinate system or an axis based on the display direction of a screen.

As in the second modification, the button 202e is used when a plurality of feature amounts are calculated in step S6 based on a plurality of directions. Referring to FIG. 14, if the button 202e is operated while "commissure" is displayed, commissure alignment in the feature amount calculation region 205 is selected. After the button 202e is selected, the button 202e may be changed to a state in which "coronary" is displayed. Alternatively, "alignment" may be displayed. In any case, if the button 202e is selected, the same or different region of interest is displayed in the display region 202b, and a display direction is set in accordance with an operation by the user. Note that a plurality of display directions may be set by the selection of the button 202e or the like. The display direction that has been set once may be recorded, and the display direction of the display region 202b may be switched in accordance with the recorded display direction. Note that a button equivalent to the button 202e may also be set in the display region 202a to allow the user to set a plurality of types of first display directions X.

The feature amount calculation region 205 displays the feature amount calculated in step S6. The feature amount calculation region 205 includes a first feature amount region 205a, a second feature amount region 205b, and a direction display region 205c.

The first feature amount region 205a displays the calculation formula for the first feature amount calculated in step S6, the first display direction X (angle) specified in step S3, and the second display direction Y1 (angle) specified in step S5. If the user applies operations to the display region 202a and the display region 202b to update the first display direction X and the second display direction Y1 in the display region 202a and the display region 202b, the display of the first feature amount region 205a is also updated based on the updated display direction. In addition, control may be performed to allow the user to update the value of the display direction X or Y1 displayed in the first feature amount region 205a to an arbitrary value by using the input interface and to, if the value is updated, update the display of the display region 202a or 202b to a display mode corresponding to a display direction corresponding to the updated value.

The second feature amount region 205b displays the calculation formula for the second feature amount calculated in the second modification, the first display direction X (angle) specified in step S3, the second display direction Y1 (angle) specified in step S5, and the third display direction Z specified in the second modification. If the user applies operations to the display region 202a and the display region 202b to update the first display direction X and the second display direction Y1 in the display region 202a and the display region 202b, the display of the second feature amount region 205b is also updated based on the updated display direction. In addition, control may be performed to allow the user to update the value of the display direction X or Y1 displayed in the second feature amount region 205b to an arbitrary value by using the input interface and to, if the value is updated, update the display of the display region 202a or 202b to a display mode corresponding to a display direction corresponding to the updated value.

Note that the two types of feature amount regions, namely, the first feature amount region 205a and the second feature amount region 205b, are used if there are two types of feature amounts to be calculated as in the second modification. That is, if there is only one type of feature amount to be calculated, the second feature amount region 205b is not necessary. In addition, if three or more types of feature amounts are calculated, a calculation formula and a display direction (angle) corresponding to each feature amount may be displayed. For example, the first feature amount region 205a and the second feature amount region 205b may be displayed side by side, and control may be performed to switch the display based on the display directions in the display regions 202a and 202b. For example, as shown in FIG. 11, if the medical image g23 including the third display direction Z is displayed in the display region 202a or 202b, the second feature amount region 205b using the third display direction Z may be displayed without displaying the first feature amount region 205a that does not use the third display direction Z.

If the calculated feature amount indicates a direction, the direction display region 205c is a region for displaying the direction upon converting it into a timepiece. That is, the direction is displayed upon being converted into a timepiece by converting and expressing one minute of the timepiece into a direction (angle) of 6°. That is, an angle of 90° is equivalent to 15 minutes indicated by the timepiece and indicates the 3 o'clock direction. Referring to FIG. 14, the solid line in the direction display region 205c is displayed at the position of 0.5 o'clock corresponding to the feature amount in the first feature amount region 205a. The chain line in the direction display region 205c is displayed at the position of 0.3 o'clock corresponding to the feature amount in the second feature amount region 205b.

According to the fourth modification described above, the processing circuitry 34 updates the first display direction X or the second display direction Y1 in accordance with a user operation and updates the display contents on the display 32 based on the updating result. The processing circuitry 34 updates the first feature amount based on the updated result. In addition to the effect of the embodiment, this makes it possible to calculate the first feature amount from the first display direction X and the second display direction Y1 in accordance with a user operation.

According to at least one of the embodiment and the modifications described above, it is possible to support surgery so as to retain a prosthetic valve at a proper angle for each patient.

The term "processor" used in the above description means a circuit such as a CPU, GPU, ASIC (Application Specific Integrated Circuit), or programmable logic device (for example, SPLD (Simple Programmable Logic Device), CPLD (Complex Programmable Logic Device), or FPGA (Field Programmable Gate Array)). If the processor is a CPU, the processor implements a function by reading out and executing a program stored in the memory. If the processor is an ASIC, the function is incorporated as a logic circuit in the circuit of the processor instead of the program being saved in the memory. Each processor in the embodiment may be implemented as a single circuit for each processor or implemented by combining a plurality of independent circuits into one processor. In addition, a plurality of constituent elements in FIG. 1 may be integrated into one processor so as to implement its function.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A medical image processing apparatus comprising:
an acquisition unit configured to acquire a three-dimensional medical image of a patient requiring retainment of a prosthetic valve;
a first region specifying unit configured to specify a first region of interest from the three-dimensional medical image;
a first direction specifying unit configured to specify a first direction of the three-dimensional medical image based on the first region of interest;
a second region specifying unit configured to specify a second region of interest from the three-dimensional medical image;
a second direction specifying unit configured to specify a second direction of the three-dimensional medical image based on the second region of interest; and
a feature amount calculation unit configured to calculate a first feature amount associated with the retainment based on the first direction and the second direction.

2. The apparatus according to claim 1, wherein the first region of interest includes a first partial region and a second partial region different from each other, and
the first region specifying unit is configured to specify the first direction based on a first positional relationship between the first partial region and the second partial region.

3. The apparatus according to claim 1 or 2, wherein the second region of interest is part of the first region of interest.

4. The apparatus according to claim 1 or 2, wherein the second region of interest is the same region as the first region of interest, and
the second region specifying unit is configured to specify the second direction based on a second positional relationship between the first partial region and the second partial region.

5. The apparatus according to claim 2, wherein the first direction specifying unit is configured to specify the first direction based on a position at which the first partial region and the second partial region indicated by the three-dimensional medical image overlap each other.

6. The apparatus according to claim 1, further comprising:
a third region specifying unit configured to specify a third region of interest different from the first region of interest and the second region of interest from the three-dimensional medical image; and
a third direction specifying unit configured to specify a third direction of the three-dimensional medical image based on the third region of interest, and
wherein the feature amount calculation unit is configured to further calculate a second feature amount based on the first direction, the second direction, and the third direction.

7. The apparatus according to claim 6, wherein the first region of interest is an aorta of the patient,
the first partial region is an ascending aorta of the patient,
the second partial region is a descending aorta of the patient,
the second region of interest is a commissure in the aortic valve of the patient,
the third region of interest is a coronary ostium,
the first feature amount is an angle at which the prosthetic valve is inserted into the patient, and
the second feature amount is an angle obtained by correcting the first feature amount so as to implement retainment in a state in which the coronary ostium is not occluded.

8. The apparatus according to claim 6, wherein the feature amount calculation unit is configured to calculate a third feature amount associated with the retainment based on a predetermined calculation formula using the first feature amount and the second feature amount as variables.

9. The apparatus according claim 1, wherein the feature amount calculation unit is configured to classify a shape of an anatomical structure in the first region of interest or the second region of interest, select a calculation formula corresponding to a result of the classification from a plurality of calculation formulae configured to calculate the first feature amount, and calculate the first feature amount based on the selected calculation formula.

10. The apparatus according to claim 1, wherein the feature amount calculation unit is configured to calculate, as the first feature amount, an angle at which the prosthetic valve is inserted into the patient.

11. The apparatus according to claim 10, wherein the feature amount calculation unit is configured to calculate, as the angle, a rotation amount of the prosthetic valve that rotates about an axis of a catheter holding the prosthetic valve.

12. The apparatus according to claim 11, wherein the angle is an angle dependent on a position of a commissure of the aortic valve of the patient, and
the rotation amount is a rotational angle for matching a commissure of the prosthetic valve with a commissure of the aortic valve.

13. The apparatus according to any one of claims 1 to 12, further comprising a display control unit configured to cause a display to display, side by side, a first display region that displays the three-dimensional medical image based on the first direction and a second display region that displays the three-dimensional medical image based on the second direction.

14. The apparatus according to claim 13, wherein the display control unit is configured to
update the first direction or the second direction in accordance with a user operation and update display contents on the display based on the updating result, and
update the first feature amount based on the updating result.

15. A medical image processing method comprising:
acquiring a three-dimensional medical image of a patient requiring retainment of a prosthetic valve;
specifying a first region of interest from the three-dimensional medical image;
specifying a first direction of the three-dimensional medical image based on the first region of interest;
specifying a second region of interest from the three-dimensional medical image;
specifying a second direction of the three-dimensional medical image based on the second region of interest; and
calculating a first feature amount associated with the retainment based on the first direction and the second direction.
